# EUROPEAN PATENT SPECIFICATION

(11) **EP 1 364 205 B1**
(45) Date of publication and mention of the grant of the patent: **23.05.2007**
(21) Application number: 02713487.3
(22) Date of filing: 28.01.2002
(51) Int. Cl.: G01N 33/00, A01K 67/027, C12N 5/00, C12N 5/02, C12N 15/00, C12N 15/09, C12N 15/63, C12N 15/70, C12N 15/74, C12N 15/85

(54) **PRODUCTION OF A MONOCLONAL ANTIBODY BY A TRANSGENIC AVIAN**
PRODUKTION EINES MONOKLONAKEN ANTIKÖRPERS DURCH EINE TRANSGENE VOGELSPEZIES
PRODUCTION D'UN ANTICORPS MONOCLONAL PAR UN OISEAU TRANSGENIQUE

(30) Priority: 02.02.2001 US 266344 P; 08.06.2001 US 877374
(43) Date of publication of application: 26.11.2003
(73) Proprietor: Avigenics, Inc., Athens, GA 30605 (US)
(72) Inventor: RAPP, Jeffrey, C., Athens, GA 30606 (US)
(74) Representative: Silveston, Judith
(86) International application number: PCT/US2002/002454
(87) International publication number: WO 2002/063293

(56) References cited:
- WO-A-00/29444
- WO-A-94/24268
- WO-A2-00/75300
- WO-A2-99/10505
- US-A- 5 804 196
- GANDOLFI F.: 'Spermatozoa, DNA binding and transgenic animals' vol. 7, May 1998, pages 147 - 155, XP001064009
- GANDOLFI F.: 'Sperm-mediated transgenesis' THERIOGENOLOGY vol. 53, January 2000, pages 127 - 137, XP002909563

## Description

### Field of the Invention

The present invention relates generally to the production of avian eggs, specifically from chickens, having heterologous antibodies therein. More specifically, the invention relates to methods of generating a transgenic avian capable of producing an egg containing a mammalian antibody capable of binding to an antigen.

### Background

The purification of a monoclonal antibody, specific for a single epitope from serum, is not feasible, since the concentration of any one antibody species in serum is so low. To overcome this practical difficulty, hybridomas were developed wherein a B lymphocyte is fused with a myeloma cell. The immortalized hybridoma cell line may be propagated indefinitely *in vivo* as ascites, or *in vitro* in tissue culture. The unique antibody synthesized by a hybridoma culture is then purified from the culture medium or ascites fluid (Kohler & Milstein, 1975, *Nature* 256: 495-497).

Monoclonal antibodies have proven of inestimable value in therapeutics, diagnostics, and as research tools because of their high specificity for target antigens. In therapeutics, monoclonal antibodies cross-linked to therapeutic agents have been targeted to specific cells. This method is of particular use in cancer treatment with chemotherapeutic agents focused against cancer cells rather than normal cells. Monoclonal antibodies are also useful for targeting and neutralizing toxic proteins or other antigens produced by microbial pathogens.

In diagnostics, monoclonal antibodies prove invaluable as tools for the detection of target enzymes, pathogens, cell specific markers, and the like. An immunoglobulin may be attached to a label for the detection of a cell, such as a cancer cell, within a patient, or alternately, form the essential component of a quantitative assay such as an enzyme-linked immunoabsorbant assay (ELISA). Monoclonal antibodies are also useful for a myriad of research applications, such as antigen detection and quantitation assays, including ELISAs and immunohistochemistry procedures.

The production of monoclonal antibodies by traditional methods, however, is labor-intensive and costly. To produce sufficient antibody once the hybridoma is isolated often requires major expenditures in tissue culture facilities or breeding of mice. In the latter case, cell transfer and ascites fluid harvesting is expensive, and still may not provide the quantities demanded for medical or industrial use.

Various strategies have been proposed to overcome the deficiencies in antibody yield, including engineering single-chain antibodies (scAb) comprising immunoglobulin heavy and light chain variable regions. The nucleic acid sequences encoding these regions are contiguous in a nucleic acid expression vector and the expressed scAb protein may be produced in bulk systems employing bacteria, yeast, plant, or animal cells. No method, however, has proven entirely satisfactory in elevating antibody yields to the levels desired for adequate commercial production. Industry, therefore, is now looking to transgenic animals that can express, for example, an exogenous protein such as an antibody under conditions that offer high yield of the protein in an active form while incorporating post-translational modifications, such as glycosylation, typically required for full functionality of the antibody.

The field of transgenics was initially developed to understand the action of a single gene in the context of the whole animal and the phenomena of gene activation, expression, and interaction. This technology has also been used to produce models for various diseases in humans and other animals and is amongst the most powerful tools available for the study of genetics, and the understanding of genetic mechanisms and function. From an economic perspective, however, the use of transgenic technology to convert animals into "protein factories" for the production of specific proteins or other substances of pharmaceutical interest (Gordon et al., 1987, *Biotechnology* 5: 1183-1187; Wilmut et al., 1990, *Theriogenology* 33: 113-123) offers significant advantages over more conventional methods of protein production by gene expression.

In this context, heterologous nucleic acids have been engineered so that an expressed protein may be joined to a protein or peptide that will allow secretion of the transgenic expression product into milk or urine, from which the protein may then be recovered. These procedures have had limited success and may require lactating animals, with the attendant costs of maintaining individual animals or herds of large species, including cows, sheep, or goats.

Historically, transgenic animals have been produced almost exclusively by microinjection of the fertilized egg. The pronuclei of fertilized eggs are microinjected *in vitro* with foreign, i.e., xenogeneic or allogeneic, heterologous DNA or hybrid DNA molecules. The microinjected fertilized eggs are then transferred to the genital tract of a pseudopregnant female (e.g., Krimpenfort et al., in U.S. Pat. Nos. 5,175,384, 5,434,340 and 5,591,669).

Microinjection techniques require equipment to handle embryos and the facility to microinject them *in vitro.* Large numbers of fertilized eggs are needed because there is a high rate of egg loss due to lysis during microinjection. Moreover, manipulated embryos are less likely to implant and survive *in utero.* Typically, 300-500 fertilized eggs must be microinjected to produce perhaps three transgenic animals. Consequently, generating large animals with these techniques is prohibitively expensive.

Genetic information also has been transferred to embryos using retroviral vectors (Jaenisch, R., 1976, *Proc. Natl. Acad. Sci. USA* 73: 1260-1264), but the technique suffers from numerous drawbacks including that the resulting animals were mosaics with different gene insertions in different tissues. (Jaenisch, R., 1980, *Cell* 19: 181-188).

An alternative method for creating a transgenic animal is nuclear replacement of fertilized ova. Totipotent, i.e., immature undifferentiated cells, are transfected *in vitro* by techniques commonly known in the art. The transfected diploid nuclei are isolated by micromanipulation and then transferred into a freshly fertilized egg, after which the "native" male and female haploid pronuclei are removed by suction. The egg cell then proceeds with embryonic development based on the transfected diploid nucleus that was moved into the ovum. However, because extreme skill is required for the micromanipulation, the technique is costly and has a low success rate.

One system that holds potential is the avian reproductive system. The production of an avian egg begins with formation of a large yolk in the ovary of the hen. The unfertilized oocyte or ovum is positioned on top of the yolk sac. After ovulation, the ovum passes into the infundibulum of the oviduct where it is fertilized, if sperm are present, and then moves into the magnum of the oviduct - lined with tubular gland cells. These cells secrete the egg-white proteins, including ovalbumin, lysozyme, ovomucoid, conalbumin and ovomucin, into the lumen of the magnum where they are deposited onto the avian embryo and yolk.

The hen oviduct offers outstanding potential as a protein bioreactor because of the high levels of protein production, the promise of proper folding and post-translation modification of the target protein, the ease of product recovery, and the shorter developmental period of chickens compared to other potential animal species. As a result, efforts have been made to create transgenic chickens expressing heterologous proteins in the oviduct by means of microinjection of DNA (PCT Publication WO 97/47739).

Bosselman *et al.,* in U.S. Patent No. 5,162,215, describes a method for introducing a replication-defective retroviral vector into a pluripotent stem cell of an unincubated chick embryo, and further describes chimeric chickens whose cells express a heterologous vector nucleic acid sequence. However, the percentage of G1 transgenic offspring (progeny from vector-positive male G0 birds) was low and varied between 1% and approximately 8%. Generally, DNA injection into avian eggs has so far led to poor and unstable transgene integration (Sang & Perry, 1989, *Mol. Reprod. Dev.* 1: 98-106) and Naito et al., 1994, *Mol. Reprod. Dev.* 37: 167-71). In addition, the use of viral vectors poses limitations on the technique, including the size of the transgene that can be incorporated into the vector and the potential for viral infection of the offspring. The production of transgenic chickens by DNA microinjection *(supra)* is both inefficient and time-consuming.

Transfer of a donor ovum to the oviduct of a recipient hen could facilitate genetic manipulation in avians. Tanaka et al., (1994, J. *Reprod. and Fertility,* 100: 447-449) produced chicks by *in vitro* fertilization (IVF) and returned the fertilized ovum to the oviduct of a recipient hen to complete the egg and shell formation. This experimental approach suggests a useful model for the production of transgenic avians.

Another method offering promise for genetic manipulation is the stable transfection of male germ cells *in vitro* and their transfer to a recipient testis. PCT Publication WO 87/05325 discloses a method of transferring organic and/or inorganic material into sperm or egg cells by using liposomes. Bachiller et al. (1991, *Mol. Reprod. Develop.* 30: 194-200) used Lipofectin-based liposomes to transfer DNA into mice sperm, and provided evidence that the liposome transfected DNA was overwhelmingly contained within the sperm's nucleus, although no transgenic mice could be produced by this technique. Nakanishi & Iritani (1993, *Mol. Reprod. Develop.* 36: 258-261) used Lipofectin-based liposomes to associate heterologous DNA with chicken sperm, which were in turn used to artificially inseminate hens. Although the heterologous DNA was detectable in many of the resultant fertilized eggs, there was no evidence of genomic integration of the heterologous DNA either in the DNA-liposome treated sperm or in the resultant chicks.

Heterologous DNA may also be transferred into sperm cells by electroporation, creating temporary, short-lived pores in the cell membrane of living cells by exposing them to a sequence of brief electrical pulses of high field strength. The pores allow cells to take up heterologous material such as DNA, while only slightly compromising cell viability. Gagne *et al.,* (1991, *Mol. Reprod. Develop.* 29: 6-15) disclosed the use of electroporation to introduce heterologous DNA into bovine sperm subsequently used to fertilize ova. However, there was no evidence of integration of the electroporated DNA either in the sperm nucleus or in the nucleus of the egg subsequent to fertilization by the sperm.

Yet another method initially developed for integrating heterologous DNA into yeast and slime molds, and later adapted to avian sperm, is restriction enzyme mediated integration (REMI) (Shemesh *et al.,* in WO 99/42569), which utilizes a linear DNA derived from a plasmid DNA by cutting that plasmid with a restriction enzyme that generates single-stranded cohesive ends. The linear, cohesive-ended DNA, together with the restriction enzyme used to produce the cohesive ends, is then introduced into the target cells by electroporation or liposome transfection. The restriction enzyme is believed to cut the genomic DNA at sites that enable the heterologous DNA to integrate via its matching cohesive ends (Schiestl and Petes, 1991, *Proc. Natl. Acad. Sci. USA* 88: 7585-7589).

Once a transgenic animal line has been created, the protein expressed from the incorporated transgene should be produced in quantities and bear any post-translational modification, such as glycosylation, that may be necessary for functionality. In the case of antibodies comprising at least two dissimilar polypeptides, the heavy and light chains must be folded to their correct tertiary conformations, cross-linked by cysteine bridges and then interact to form at least one antigen-binding site. Preferably, the antigen-binding antibody should be produced in the white of an avian egg from which it may be readily purified. The economic advantage of breeding flocks of transgenic birds laying eggs expressing active and functional monoclonal antibodies would be significant when compared to more traditional animals, such as the cow, producing a heterologous protein in milk.

What is needed, therefore, is a method of introducing a transgene into an avian, such as a chicken, that will encode and express an antibody capable of binding an antigen in the white of a hard-shelled egg.

What is particularly needed are methods of expressing a functional antibody in an avian egg, preferably a chicken egg. What is also needed is for variable regions of immunoglobulin heavy and light chain polypeptides, expressed individually or together, to combine in an avian egg or after isolation of the individual polypeptides thereby forming an active antibody.

What is further needed are methods of generating a transgenic bird, preferably a chicken, capable of expressing in the white of the egg of the transgenic bird an antibody capable of selectively binding an antigen, or immunoglobulin polypeptides that may combine, either in an egg or after isolation of the polypeptides therefrom, to form an active antibody.

### Summary of the Invention

Briefly described, the present invention relates to novel methods of producing an avian egg, preferably a chicken egg, having a heterologous antibody protein therein that is capable of binding to an antigen. More specifically, the present invention relates to methods of producing transgenic avians, preferably chickens, wherein the incorporated transgene is expressed to give a constituent protein of the white of a hard-shell egg.

The transgene incorporated into the genomic DNA of a recipient bird encodes at least one immunoglobulin polypeptide that may be an immunoglobulin heavy chain, a light chain or the variable regions thereof. The nucleic acid encoding the immunoglobulin polypeptides may be operatively linked to a transcription promoter and a transcription terminator.

The present invention further relates to nucleic acid vectors and transgenes derived therefrom that incorporate immunoglobulin polypeptide-encoding regions, wherein a first polypeptide-encoding region is operatively linked to a transcription promoter and a second polypeptide-encoding region is operatively linked to an Internal Ribosome Entry Sequence (IRES). This nucleic acid construct, when inserted into the genome of a bird and expressed therein, will generate individual immunoglobulin polypeptides that may be post-translationally modified and combined in the white of a hard-shell bird egg to form an antibody capable of binding to an antigen. Alternatively, the expressed polypeptides may be isolated from an avian egg and combined *in vitro* to form a functional antibody.

The present invention also relates to methods that use expression vectors of viral or plasmid origin. The transcriptional promoters therein may be tissue-specific so that the immunoglobulin polypeptides encoded by the expression vectors may be expressed as a protein constituent of the white of an avian egg.

The present invention provides methods for the introduction into an avian genome of at least one transgene encoding at least one immunoglobulin polypeptide. These methods include sperm-mediated transfer, whereby transgenic genes are incorporated into avian sperm by liposomes, electroporation, restriction enzyme mediated integration (REMI), and similar methods. The modified sperm may then be returned to the testis of a male bird that may then be mated with a female, thereby generating transgenic offspring. In an alternate embodiment of the present invention, the modified sperm may be used directly to fertilize the female bird by artificial insemination to generate transgenic offspring.

The present invention provides methods for further incorporating a transgene into the nucleus of an avian cell cultured *in vitro.* The transgenic cell nucleus may then be transferred to a fertilized enucleated cell. The enucleated cell may be an embryonic cell of a bird egg visualized through overlying yolk or tissue by using two photon laser scanning microscopy.

Additional objects and aspects of the present invention will become more apparent upon review of the detailed description set forth below when taken in conjunction with the accompanying figures, which are briefly described as follows.

### Brief Description of the Figures

Fig. I. illustrates human monoclonal antibody expression by cultured quail oviduct cells. Cells were transfected with pCMV-EGFP (negative control), p1086 (L-chain), p1083 (H-chain) or p1083 and p1086. p1086 and p1083 contained the cytomegalovirus (CMV) immediate early enhancer/promoter which controlled expression of the antibody light chains and heavy chains, respectively. Samples were assayed for light and heavy chain content by ELISA and FACS.

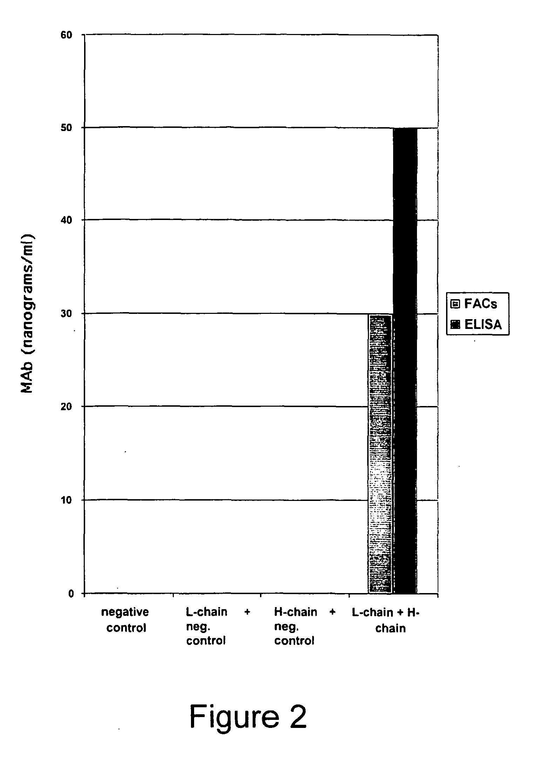

Fig. 2. illustrates human monoclonal antibody expression by cultured chicken whole embryo fibroblasts (WEFs) transfected with heavy and light chain cDNAs. Cells were transfected with pCMV-EGFP (negative control), p1086 (L-chain), or p1083 (H-chain) or co-transfected with both plasmids carrying the light and heavy chains respectively. p1086 and p1083 contained the CMV immediate early enhancer/promoter which controlled expression of the light chain and heavy chain, respectively. Samples were assayed using a fluorescence-activated cell sorting (FACS) method and by ELISA.

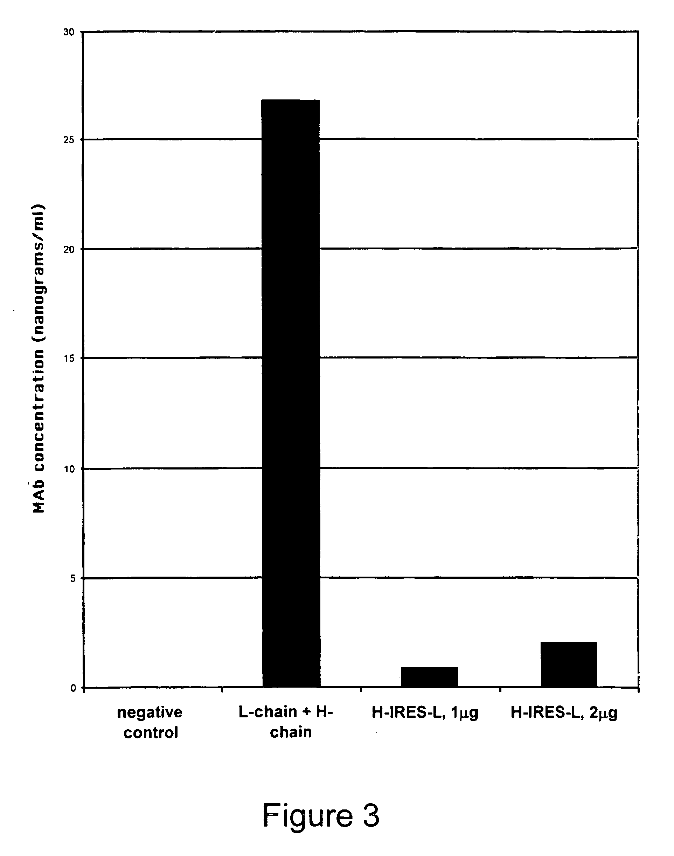

Fig. 3. illustrates human monoclonal antibody expression by cultured chicken whole embryo fibroblasts (WEFs) transfected with an IRES vector. Cells were transfected with pCMV-EGFP alone (negative control), cotransfected with p1086 (L-chain) and p1083 (H-chain), or transfected with either 1 µg or 2 µg of pCMV-L chain-IRES-H chain (L-IRES-H). p1086 and p1083 included the CMV immediate early enhancer/promoter which controlled expression of the light chain or heavy chain, respectively. pCMV-L chain-IRES-H chain contained the CMV immediate early enhancer/promoter which controlled expression of the heavy and light chains as a single transcript, with an IRES element positioned downstream from the light chain cDNA sequence and upstream from the heavy chain cDNA sequence. L-IRES-H #1: transfection contained 1 µg of plasmid DNA; L-IRES-H #2: contained 2 µg of plasmid DNA. Samples were assayed by ELISA.

### Detailed Description of the Preferred Embodiments

Reference now will be made in detail to the presently preferred embodiments of the invention, one or more examples of which are illustrated in the accompanying drawings. Each example is provided by way of explanation of the invention, not limitation of the invention. In fact, it will be apparent to those skilled in the art that various modifications, combinations, additions, deletions and variations can be made in the present invention without departing from the scope or spirit of the invention. For instance, features illustrated or described as part of one embodiment can be used in another embodiment to yield a still further embodiment. It is intended that the present invention cover such modifications, combinations, additions, deletions and variations as fall within the scope of the appended claims and their equivalents.

This description uses gene nomenclature accepted by the Cucurbit Genetics Cooperative as it appears in the *Cucurbit Genetics Cooperative Report* 18:85 (1995), herein incorporated by reference in its entirety. Using this gene nomenclature, genes are symbolized by italicized Roman letters. If a mutant gene is recessive to the normal type, then the symbol and name of the mutant gene appear in italicized lower case letters.

Throughout this application various publications are referenced. The disclosures of these publications are hereby incorporated by reference in their entireties in this application to more fully describe the state of the art to which this invention pertains.

For convenience, certain terms employed in the specification, examples, and appended claims are collected here.

### Definitions

The term "animal" as used herein refers to all vertebrate animals, including humans and birds. It also includes an individual animal in all stages of development, including embryonic and fetal stages.

The term "avian" as used herein refers to any species, subspecies or race of organism of the taxonomic class *aves,* such as, but not limited to, such species as chicken, turkey, duck, goose, quail, pheasants, parrots, finches, hawks, crows and ratites including ostrich, emu and cassowary. The term includes the various known strains of *Gallus gallus,* or chickens, (for example, White Leghorn, Brown Leghorn, Barred-Rock, Sussex, New Hampshire, Rhode Island, Ausstralorp, Minorca, Amrox, California Gray, Italian Partidge-colored), as well as strains of turkeys, pheasants, quails, duck, ostriches and other poultry commonly bred in commercial quantities.

The term "nucleic acid" as used herein refers to any natural and synthetic linear and sequential arrays of nucleotides and nucleosides, for example cDNA, genomic DNA, mRNA, tRNA, oligonucleotides, oligonucleosides and derivatives thereof. For ease of discussion, such nucleic acids may be collectively referred to herein as "constructs," "plasmids," or "vectors." Representative examples of the nucleic acids of the present invention include bacterial plasmid vectors including expression, cloning, cosmid and transformation vectors such as, but not limited to, pBR322, animal viral vectors such as, but not limited to, modified adenovirus, influenza virus, adeno-associated virus, polio virus, pox virus, retrovirus, and the like, vectors derived from bacteriophage nucleic acid, and synthetic oligonucleotides like chemically synthesized DNA or RNA. The term "nucleic acid" further includes modified or derivatised nucleotides and nucleosides such as, but not limited to, halogenated nucleotides such as, but not only, 5-bromouracil, and derivatised nucleotides such as biotin-labeled nucleotides.

The terms "polynucleotide", "oligonucleotide", and "nucleic acid sequence" are used interchangeably herein and include, but are not limited to, coding sequences (polynucleotide(s) or nucleic acid sequence(s) which are transcribed and translated into polypeptide *in vitro* or *in vivo* when placed under the control of appropriate regulatory or control sequences), control sequences (e.g., translational start and stop codons, promoter sequences, ribosome binding sites, polyadenylation signals, transcription factor binding sites, transcription termination sequences, upstream and downstream regulatory domains, enhancers, silencers, and the like), and regulatory sequences (DNA sequences to which a transcription factor(s) binds and alters the activity of a gene's promoter either positively (induction) or negatively (repression)). No limitation as to length or synthetic origin are suggested by the terms described herein.

The term "isolated nucleic acid" as used herein refers to a nucleic acid with a structure (a) not identical to that of any naturally occurring nucleic acid or (b) not identical to that of any fragment of a naturally occurring genomic nucleic acid spanning more than three separate genes, and includes DNA, RNA, or derivatives or variants thereof. The term covers, for example, (a) a DNA which has the sequence of part of a naturally occurring genomic molecule but is not flanked by at least one of the coding sequences that flank that part of the molecule in the genome of the species in which it naturally occurs; (b) a nucleic acid incorporated into a vector or into the genomic nucleic acid of a prokaryote or eukaryote in a manner such that the resulting molecule is not identical to any vector or naturally occurring genomic DNA; (c) a separate molecule such as a cDNA, a genomic fragment, a fragment produced by polymerase chain reaction (PCR), ligase chain reaction (LCR) or chemical synthesis, or a restriction fragment; (d) a recombinant nucleotide sequence that is part of a hybrid gene, i.e., a gene encoding a fusion protein, and (e) a recombinant nucleotide sequence that is part of a hybrid sequence that is not naturally occurring. Isolated nucleic acid molecules of the present invention can include, for example, natural allelic variants as well as nucleic acid molecules modified by nucleotide deletions, insertions, inversions, or substitutions such that the resulting nucleic acid molecule still essentially encodes an immunoglobulin or a variant thereof

As used herein the terms "polypeptide" and "protein" refer to a polymer of amino acids of three or more amino acids in a serial array, linked through peptide bonds. The term "polypeptide" includes proteins, protein fragments, protein analogues, oligopeptides and the like. The term "polypeptide" contemplates polypeptides as defined above that are encoded by nucleic acids, produced through recombinant technology, isolated from an appropriate source such as a bird, or are synthesized. The term "polypeptide" further contemplates polypeptides as defined above that include chemically modified amino acids or amino acids covalently or noncovalently linked to labeling ligands.

The term "fragment" as used herein to refer to a nucleic acid (e.g., cDNA) refers to an isolated portion of the subject nucleic acid constructed artificially (e.g., by chemical synthesis) or by cleaving a natural product into multiple pieces, using restriction endonucleases or mechanical shearing, or a portion of a nucleic acid synthesized by PCR, DNA polymerase or any other polymerizing technique well known in the art, or expressed in a host cell by recombinant nucleic acid technology well known to one of skill in the art. The term "fragment" as used herein may also refer to an isolated portion of a polypeptide, wherein the portion of the polypeptide is cleaved from a naturally occurring polypeptide by proteolytic cleavage by at least one protease, or is a portion of the naturally occurring polypeptide synthesized by chemical methods well known to one of skill in the art.

The term "gene" or "genes" as used herein refers to nucleic acid sequences (including both RNA or DNA) that encode genetic information for the synthesis of a whole RNA, a whole protein, or any portion of such whole RNA or whole protein. Genes that are not naturally part of a particular organism's genome are referred to as "foreign genes", "heterologous genes" or "exogenous genes" and genes that are naturally a part of a particular organism's genome are referred to as "endogenous genes." The term "gene product" refers to RNAs or proteins that are encoded by the gene. "Foreign gene products" are RNA or proteins encoded by foreign genes and "endogenous gene products" are RNA or proteins encoded by endogenous genes. "Heterologous gene products" are RNAs or proteins encoded by foreign, heterologous or heterologous genes and, therefore, are not naturally expressed in the cell.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from the RNA nucleic acid molecule to yield a protein or polypeptide or a portion thereof.

The term "transcription regulatory sequences" as used herein refers to nucleotide sequences that are associated with a gene nucleic acid sequence and that regulate the transcriptional expression of the gene. The "transcription regulatory sequences" may be isolated and incorporated into a vector nucleic acid to enable regulated transcription in appropriate cells of portions of the vector DNA. The "transcription regulatory sequences" may precede, but are not limited to, the region of a nucleic acid sequence that is in the region 5' of the end of a protein coding sequence that may be transcribed into mRNA. Transcriptional regulatory sequences may also be located within a protein coding region, in regions of a gene that are identified as "intron" regions, or may be in regions of nucleic acid sequence that are in the region of nucleic acid.

The term "coding region" as used herein refers to a continuous linear arrangement of nucleotides that may be translated into a protein. A full length coding region is translated into a full length protein; that is, a complete polypeptide as would be translated in its natural state absent any post-translational modifications. A full length coding region may also include any leader protein sequence or any other region of the protein that may be excised naturally from the translated protein.

The term "cohesive end" as used herein refers to regions at the termini of nucleic acid molecules that can form specific interactions with one another. Cohesive ends of nucleic acids may be generated by restriction endonuclease cleavage of a double strand nucleic acid. In the specific interactions, an adenine base within one strand of a nucleic acid can form two hydrogen bonds with thymine within a second nucleic acid strand when the two nucleic acid strands are in opposing polarities. Also in the specific interactions, a guanine base within one strand of a nucleic acid can form three hydrogen bonds with cytosine within a second nucleic acid strand when the two nucleic acid strands are in opposing polarities. Complementary nucleic acids as referred to herein may further comprise modified bases wherein a modified adenine may form hydrogen bonds with a thymine or modified thymine, and a modified cytosine may form hydrogen bonds with a guanine or a modified guanine.

The terms "nucleic acid vector" or "vector" as used herein refer to a natural or synthetic single or double stranded plasmid or viral nucleic acid molecule that can be transfected or transformed into cells and replicate independently of, or within, the host cell genome. A circular double stranded plasmid can be linearized by treatment with an appropriate restriction enzyme based on the nucleotide sequence of the plasmid vector. A nucleic acid can be inserted into a vector by cutting the vector with restriction enzymes and ligating the pieces together. The nucleic acid molecule can be RNA or DNA.

The term "plasmid" as used herein refers to a small, circular DNA vector capable of independent replication within a bacterial or yeast host cell.

The term "expression vector" as used herein refers to a nucleic acid vector that may further include at least one regulatory sequence operably linked to a nucleotide sequence coding for the an immunoglobulin polypeptide. Regulatory sequences are well recognized in the art and may be selected to ensure good expression of the linked nucleotide sequence without undue experimentation by those skilled in the art. As used herein, the term "regulatory sequences" includes promoters, enhancers, and other elements that may control expression. Standard molecular biology textbooks such as Sambrook *et al.* eds "Molecular Cloning: A Laboratory Manual" 2nd ed. Cold Spring Harbor Press (1989) and Lodish *et al.,* eds., "Molecular Cell Biology," Freeman (2000) and incorporated herein by reference in their entireties, may be consulted to design suitable expression vectors, promoters, and other expression control elements. It should be recognized, however, that the choice of a suitable expression vector depends upon multiple factors including the choice of the host cell to be transformed and/or the type of protein to be expressed. Also useful for various applications are tissue-selective (i.e., tissue-specific) promoters, i.e., promoters from which expression occurs preferentially in cells of a particular kind of tissue, compared to one or more other types of tissue. An exemplary tissue-specific promoter is a chicken oviduct-specific promoter that is naturally associated with the proteins of avian egg whites including ovalbumin, lysozyme, ovomucoid, conalbumin and ovomucin and the like.

Useful promoters also include exogenously inducible promoters. These are promoters that can be "turned on" in response to an exogenously supplied agent or stimulus, which is generally not an endogenous metabolite or cytokine. Examples include an antibiotic-inducible promoter, such as a tetracycline-inducible promoter, a heat-inducible promoter, a light-inducible promoter, or a laser inducible promoter. (e.g., Halloran *et al.,* 2000, *Development* 127(9): 1953-1960; Gemer *et al.,* 2000, *Int. J. Hyperthermia* 16(2): 171-81; Rang and Will, 2000, *Nucleic Acids Res.* 28(5): 1120-5; Hagihara *et al.,* 1999, *Cell Transplant.* 8(4): 4314; Huang *et al.,* 1999, *Mol. Med.* 5(2): 129-37; Forster, *et al.,* 1999, *Nucleic Acids Res.* 27(2): 708-10; and Liu *et al.,* 1998, *Biotechniques* 24(4): 624-8, 630-2 (1998), incorporated herein by reference in their entireties).

The terms "IRES" and "internal ribosome entry site" as used herein refer to a region of a nucleic acid, most typically an RNA molecule, wherein eukaryotic initiation of protein synthesis occurs far downstream of the 5' end of the RNA molecule. A 43S preinitiation complex comprising the elf2 protein bound to GTP and Met-tRNAᵢ^{Met}, the 40S ribosomal subunit, and faction elf3 and elf1A may bind to an "IRES" before locating an AUG start codon. An "IRES" may be used to initiate translation of a second coding region downstream of a first coding region, wherein each coding region is expressed individually, but under the initial control of a single upstream promoter. An "IRES" may be located in a viral RNA or a eukaryotic cellular mRNA.

The term "cytoplast" as used herein refers to a chromosome-free recipient cell, wherein chromosomal removal is referred to as enucleation when the nucleus of a cell is removed or destroyed.

The terms "transformation" and "transfection" as used herein refer to the process of inserting a nucleic acid into a host, preferably a cell. Many techniques are well known to those skilled in the art to facilitate transformation or transfection of a nucleic acid into a prokaryotic or eukaryotic organism. These methods involve a variety of techniques including, but not limited to, treating the cells with high concentrations of salt such as, but not only, a calcium or magnesium salt, an electric field, detergent, or liposome mediated transfection, to render the host cell competent for the uptake of the nucleic acid molecules, and by such methods as sperm-mediated and restriction-mediated integration.

The term "recombinant cell" refers to a cell that has a new combination of nucleic acid segments that are not covalently linked to each other in nature. A new combination of nucleic acid segments can be introduced into an organism using a wide array of nucleic acid manipulation techniques available to those skilled in the art. A recombinant cell can be a single eukaryotic cell, or a single prokaryotic cell, or a mammalian cell. The recombinant cell can harbor a vector that is extragenomic. An extragenomic nucleic acid vector does not insert into the cell's genome. A recombinant cell can further harbor a vector or a portion thereof that is intragenomic. The term "intragenomic" defines a nucleic acid construct incorporated within the recombinant cell's genome.

The term "recombinant nucleic acid" as used herein refers to combinations of at least two nucleic acid sequences that are not naturally found in a eukaryotic or prokaryotic cell. The nucleic acid sequences may include, but are not limited to nucleic acid vectors, gene expression regulatory elements, origins of replication, sequences that when expressed confer antibiotic resistance, and protein-encoding sequences. The term "recombinant polypeptide" is meant to include a polypeptide produced by recombinant DNA techniques such that it is distinct from a naturally occurring polypeptide either in its location, purity or structure. Generally, such a recombinant polypeptide will be present in a cell in an amount different from that normally observed in nature.

As used herein, the term "epitope" refers to a part of the protein that can specifically bind to an antibody by fitting into the antigen-binding site of the antibody.

The term "antibody" as used herein refers to polyclonal and monoclonal antibodies and fragments thereof, and immunologic binding equivalents thereof. The term "antibody" refers to a homogeneous molecular entity, or a mixture such as a polyclonal serum product made up of a plurality of different molecular entities, and may further comprise any modified or derivatised variant thereof that retains the ability to specifically bind an epitope. A monoclonal antibody is capable of selectively binding to a target antigen or epitope.

The term "immunoglobulin polypeptide" as used herein refers to a polypeptide derived from a constituent polypeptide of an antibody. An "immunological polypeptide" may be, but is not limited to, an immunological heavy or light chain and may include a variable region, a diversity region, joining region and a constant region or any combination, variant or truncated form thereof. The term "immunological polypeptides" further includes single-chain antibodies comprised of, but not limited to, an immunoglobulin heavy chain variable region, an immunoglobulin light chain variable region and optionally a peptide linker.

Described herein are methods for the production of cells generating antibodies capable of specifically recognizing one or more differentially expressed or pathway gene epitopes and methods of isolating from the cells nucleic acids that encode the native immunoglobulin polypeptides and which may be used to incorporate into expression vectors, transfection vectors and used to generate animals according to the present invention. The isolated nucleic acids may also be used in techniques known to those skilled in the art to generate recombinant nucleic acids.

For the production of serum cells generating antibodies to selectively bind to an antigen bearing epitopes, various host animals may be immunized by injection with the target protein, or a portion thereof. Such host animals may include but are not limited to humans, rabbits, mice, and rats, to name a few. Various adjuvants may be used to increase the immunologic response, depending on the host species, including, but not limited to, Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.*

Polyclonal antibodies are heterogeneous populations of antibody molecules derived from the sera of animals immunized with an antigen, such as a target gene product, or an antigenic functional derivative thereof. Monoclonal antibodies, which are homogeneous populations of antibodies to a particular antigen, may be obtained by any technique that provides for the production of antibody molecules by continuous cell lines in culture. These include, but are not limited to the hybridoma technique of Kohler and Milstein, 1975, *Nature* 256: 495-497, and U.S. Patent No. 4,376,110; the human B-cell hybridoma technique (Kosbor *et al.,* 1983, *Immunology Today* 4: 72; Cole *et al.,* 1983, *Proc. Natl. Acad. Sci.* 80: 2026-2030, and the EBV-hybridoma technique (Cole *et al.,* 1985, in "Monoclonal Antibodies And Cancer Therapy," Alan R. Liss, Inc. pp. 77-96). Briefly, spleen cells are harvested from an immunized mouse and fused with immortalizing cells (i.e., myeloma cells) to yield antibody-producing hybridomas. Hybridomas can be screened immunochemically for the production of monoclonal antibodies specifically reactive with the antigen.

In addition, techniques developed for the production of "chimeric antibodies" (Morrison *et al.,* 1984, *Proc. Natl. Acad. Sci.* 81: 6851-6855; Neuberger *et al.,* 1984, *Nature* 312: 604-608; Takeda *et al.,* 1985, *Nature* 314: 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Alternatively, or in addition, the constant domains of an immunoglobulin variable region from one species may be exchanged with the corresponding regions of a second species.

Alternatively, techniques for the production of single chain antibodies described in, for example, U.S. Patent No. 4,946,778; Bird, 1988, *Science* 242: 423-426; Huston *et al.,* 1988, *Proc. Natl. Acad. Sci.* 85: 5879-5883; and Ward *et al.,* 1989, *Nature* 334: 544-546 can be adapted to produce differentially expressed or pathway gene-single chain antibodies. Single chain antibodies are formed by linking the heavy and light chain fragments of the Fv region via an amino acid bridge, resulting in a single chain polypeptide.

Once available, the cells may be used to provide isolated nucleic acids encoding constituent polypeptides of the antibody of interest and which may be obtained for insertion into vectors. Suitable cloning techniques are described, for example, in Sambrook *et al.* eds "Molecular Cloning: A Laboratory Manual" 2nd ed. Cold Spring Harbor Press (1989). Antibodies may include, but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

"Gene delivery (or transfection) mixture," in the context of the methods of sperm mediated transfer described herein, refers to selected genetic material, for example, with an effective amount of lipid transfecting agent, for example, a cationic or polycationic lipid, such as polybrene. The amount of each component of the mixture is chosen so that the genetic modification, by transfection or transduction for example, of a specific species of cell is optimized. Such optimization requires no more than routine experimentation. The ratio of DNA to lipid is broad, preferably about 1:1, although other proportions can also be utilized depending on the type of lipid transfecting agent used.

The term "transfecting agent" as used herein refers to a composition of matter added to the genetic material for enhancing the uptake of heterologous DNA segment(s) into a eukaryotic cell, preferably an avian cell, and more preferably a chicken germ cell. The enhancement is measured relative to the uptake in the absence of the transfecting agent. Examples of transfecting agents include adenovirus-transferrin-polylysine-DNA complexes. These complexes generally augment the uptake of DNA into the cell and reduce its breakdown during its passage through the cytoplasm to the nucleus of the cell. These complexes can be targeted to the male germ cells using specific ligands that are recognized by receptors on the cell surface of the germ cell, such as the c-kit ligand or modifications thereof.

Other preferred transfecting agents include but are not limited to lipofectin, lipfectamine, DIMRIE C, Supeffect, and Effectin (Qiagen), unifectin, maxifectin, DOTMA, DOGS (Transfectam; dioctadecylamidoglycylspermine), DOPE (1,2-dioleoyl-sn-glycero-3-phosphoethanolamine), DOTAP (1,2-dioleoyl-3-trimethylammonium propane), DDAB (dimethyl dioctadecytammonium bromide), DHDEAB (N,N-di-n-hexadecyl-N,N-dihydroxyethyl ammonium bromide), HDEAB (N-n-hexadecyIN,N-dihydroxyethylammonium bromide), polybrene, or poly(ethylenimine) (PEI) and the like. These non-viral agents have the advantage that they facilitate stable integration of xenogenic DNA sequences into the vertebrate genome, without size restrictions commonly associated with virus-derived transfecting agents.

The term "male germ cells" as used herein refers to spermatozoa (i.e., male gametes) and developmental precursors thereof. In fetal development, primordial germ cells are thought to arise from the embryonic ectoderm, and are first seen in the epithelium of the endodermal yolk sac at the E8 stage. From there they migrate through the hindgut endoderm to the genital ridges. In the sexually mature male vertebrate animal, there are several types of cells that are precursors of spermatozoa, and which can be genetically modified, including the primitive spermatogonial stem cells, known as A0/As, which differentiate into type B spermatogonia. The latter further differentiate to form primary spermatocytes, and enter a prolonged meiotic prophase during which homologous chromosomes pair and recombine. Useful precursor cells at several morphological/developmental stages are also distinguishable: preleptotene spermatocytes, leptotene spermatocytes, zygotene spermatocytes, pachytene spermatocytes, secondary, spermatocytes, and the haploid spermatids. The latter undergo further morphological changes during spermatogenesis, including the reshaping of their nucleus, the formation of aerosome, and assembly of the tail. The final changes in the spermatozoon (i.e., male gamete) take place in the genital tract of the female, prior to fertilization.

The term "transgenic animal" as used herein refers to any animal, preferably an avian species, most preferably a chicken, in which one or more of the cells of the bird contain heterologous nucleic acid introduced by way of human intervention, such as by transgenic techniques well known in the art. The nucleic acid is introduced into a cell, directly or indirectly by introduction into a precursor of the cell, by way of deliberate genetic manipulation, such as by sperm-mediated or restriction-enzyme mediated integration, microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extrachromosomally replicating DNA. In the typical transgenic animal, the transgene causes cells to express a recombinant form of an immunoglobulin polypeptide or a variant polypeptide thereof.

The terms "chimeric animal" or "mosaic animal" are used herein to refer to animals in which the recombinant gene is found, or in which the recombinant is expressed in some but not all cells of the animal. The term "tissue-specific chimeric animal" indicates that the gene is present and expressed in some tissues, but not others.

As used herein, the term "transgene" means a nucleic acid sequence (encoding, for example, an immunoglobulin heavy chain, an immunoglobulin light chain or fragments thereof, that is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of a selected nucleic acid.

The terms "ovum" and "oocyte" are used interchangeably herein. Although only one ovum matures at a time, an animal is born with a finite number of ova. In avian species, such as a chicken, ovulation, which is the shedding of an egg from the ovarian follicle, occurs when the brain's pituitary gland releases a luteinizing hormone. Mature follicles form a stalk or pedicle of connective tissue and smooth muscle. Immediately after ovulation the follicle becomes a thin-walled sac, the post-ovulatory follicle. The mature ovum erupts from its sac and starts its journey through the oviduct. Eventually, the ovum enters the infundibulum where fertilization occurs. Fertilization must take place within 15 minutes of ovulation, before the ovum becomes covered by albumen. During fertilization, sperm (avians have polyspermic fertilization) penetrate the blastodisc. When the sperm lodges within this germinal disk, an embryo begins to form as a "blastoderm" or "zygote."

The term "donor cell" is used herein to describe the source of the nuclear structure that is transplanted to the recipient enucleated cytoplast. All cells of normal karyotype, including embryonic, fetal, and adult somatic cells, and further including cells in a quiescent state, may be nuclear donors. The use of non-quiescent cells as nuclear donors has been described by Cibelli, *et al.,* 1998, *Science* 280: 1256 - 8.

The term "recipient cell" as used herein refers to the enucleated recipient cell, including but not limited to an enucleated metaphase I or 11 oocyte, an enucleated unactivated oocyte, or an enucleated preactivated oocyte. Enucleation may be accomplished by splitting the cell into halves; by aspirating the metaphase plate, pronucleus, or pronuclei; by irradiation; or by any means known to one of ordinary skill in the art that provides a recipient cell no longer containing functional nuclear genetic material while remaining suitable for accepting donor genetic material. For example, one suitable means for enucleating a cell according to the present invention is two-photon laser-mediated ablation ("TPLSM"), which is further useful to guide mechanical enucleation.

The term "knock-in animal" refers to an animal that carries a specific nucleic acid sequence such as a "knock-in sequence" in a predetermined coding or noncoding region, wherein the knock-in sequence is introduced through methods of recombination, such as homologous recombination. The recombination event comprises replacing all or part of a gene of the animal by a functional homologous gene or gene segment of another animal, where the respective knock-in sequence is placed in the genomic sequence.

### Abbreviations

Abbreviations used in the present specification include the following: aa, amino acid(s); bp, base pair(s); cDNA, DNA complementary to RNA; mRNA, messenger RNA; tRNA, transfer RNA; nt, nucleotide(s); SSC, sodium chloride-sodium citrate; DMSO, dimethyl sulfoxide; TPLSM, two photon laser scanning microscopy; REMI, restriction enzyme mediated integration; V region, immunoglobulin variable region; D region, immunoglobulin diversity region; J region, immunoglobulin joining region; C region, immunoglobulin constant region; mAb, monoclonal antibody; WEFs, whole embryo fibroblasts.

### Recombinant immunoglobulin-derived nucleic acids and expression thereof

Nucleic acid molecules encoding immunoglobulin polypeptides of the present invention can be incorporated into cells using conventional recombinant DNA technology. The nucleic acid molecule encoding an antibody or a fragment thereof, may be inserted into an expression system to which the DNA molecule is heterologous (i.e. not normally present). For expression in heterologous systems, the heterologous DNA molecule is inserted into the expression system or vector in proper sense orientation and correct reading frame. The vector contains the necessary elements for the transcription and translation of the inserted protein-coding sequences.

U.S. Patent No. 4,237,224 to Cohen and Boyer, which is hereby incorporated by reference in its entirety, describes the production of expression systems in the form of recombinant plasmids using restriction enzyme cleavage and ligation with DNA ligase. These recombinant plasmids are then introduced by means of transformation and replicated in unicellular cultures including prokaryotic organisms and eukaryotic cells grown in tissue culture. Moreover, it is contemplated to be within the scope of the present invention for the vector to be any suitable vector known to those of skill in the art such as viral vectors including viral expression vectors.

Antibody-related nucleic acid sequences or derivative or truncated variants thereof, may be introduced into viruses such as *Vaccinia* virus. Methods for making a viral recombinant vector useful for expressing an immunoglobulin polypeptide are analogous to the methods disclosed in U.S. Patent Nos. 4,603,112; 4,769,330; 5,174,993; 5,505,941; 5,338,683; 5,494,807; 4,722,848; Paoletti, E., 1996, *Proc. Natl. Acad. Sci.* 93: 11349-11353; Moss, B., 1996, *Proc. Natl. Acad. Sci.* 93: 11341-11348; Roizman, *Proc. Natl. Acad Sci. 93:* 11307-11302; Frolov *et al.,* 1996, *Proc. Natl. Acad. Sci.* 93: 11371-11377; Grunhaus *et al.,* 1993, in Seminars in Virology 3: 237-252 and U.S. Patent Nos. 5,591,639; 5,589,466; and 5,580,859 relating to DNA expression vectors, *inter alia;* the contents of which are incorporated herein by reference in their entireties.

Recombinant viruses can also be generated by transfection of plasmids into cells infected with virus. Suitable vectors include, but are not limited to, viral vectors such as lambda vector system λgt11, λgt WES.tB, Charon 4, and plasmid vectors such as pBR322, pBR325, pACYC177, pACYC184, pUC8, pUC9, pUC18, pUC19, pLG339, pR290, pKC37, pKC101, SV 40, pBluescript II SK +/- or KS +/- and any derivatives thereof. Recombinant molecules can be introduced into cells via transformation, particularly transduction, conjugation, mobilization, or electroporation. The DNA sequences are cloned into the vector using standard cloning procedures in the art, as described by Maniatis *et al.,* 1982, in Molecular Cloning: A Laboratory Manual, Cold Springs Laboratory, Cold Springs Harbor, N.Y., the contents of which is hereby incorporated by reference in its entirety.

Different genetic signals and processing events control many levels of gene expression (e.g., DNA transcription and messenger RNA (mRNA) translation). Transcription of DNA is dependent upon the presence of a promoter that is a DNA sequence that directs the binding of RNA polymerase and thereby promotes mRNA synthesis.

Once the isolated DNA molecule of the present invention has been cloned into an expression system, it is ready to be incorporated into a host cell. Such incorporation can be carried out by the various forms of transformation noted above, depending upon the vector/host cell system.

Recombinant expression vectors can be designed for the expression of the encoded proteins in eukaryotic cells. Useful vectors may comprise constitutive or inducible promoters to direct expression of either fusion or non-fusion proteins. With fusion vectors, a number of amino acids are usually added to the expressed target gene sequence such as, but not limited to, a protein sequence for thioredoxin. A proteolytic cleavage site may further be introduced at a site between the target recombinant protein and the fusion sequence. Additionally, a region of amino acids such as a polymeric histidine region may be introduced to allow binding of the fusion protein to metallic ions such as nickel bonded to a solid support, and thereby allow purification of the fusion protein. Once the fusion protein has been purified, the cleavage site allows the target recombinant protein to be separated from the fusion sequence. Enzymes suitable for use in cleaving the proteolytic cleavage site include, but are not limited to, Factor Xa and thrombin. Fusion expression vectors that may be useful in the present invention include pGex (Amrad Corp., Melbourne, Australia), pRlT5 (Pharmacia, Piscataway, NJ) and pMAL (New England Biolabs, Beverly, MA), that fuse glutathione S-transferase, protein A, or maltose E binding protein, respectively, to the target recombinant protein.

Expression of a foreign gene can be obtained using eukaryotic host cells such as avian cells. The use of eukaryotic host cells permit partial or complete post-translational modification such as, but not only, glycosylation and/or the formation of the relevant inter- or intra-chain disulfide bonds. Examples of vectors useful for expression in the chicken *Gallus gallus* include pYepSecl as in Baldari *et al.,* 1987, E.M.B.O. 6: 229-234, the contents of which is incorporated herein by reference in its entirety, and commercial vectors such as pYES2 (Invitrogen Corp., San Diego, CA),

### Viral host cell transformation

A preferred approach for *in vivo* introduction of nucleic acid encoding one of the subject immunoglobulin polypeptides into a cell is by use of a viral vector containing nucleic acid, e.g. a cDNA, encoding the gene product. Infection of cells with a viral vector has the advantage that a large proportion of the targeted cells can receive the nucleic acid. Additionally, molecules encoded within the viral vector, e.g., by a cDNA contained in the viral vector, are expressed efficiently in cells that have taken up viral vector nucleic acid.

Retrovirus vectors and adeno-associated virus vectors are generally understood to be the recombinant gene delivery system of choice for the transfer of heterologous genes *in vivo.* These vectors provide efficient delivery of genes into cells, and the transferred nucleic acids are stably integrated into the chromosomal DNA of the host. Recombinant retrovirus can be constructed wherein the retroviral coding sequences (*gag, pol, env* for example) have been replaced by nucleic acid encoding an immunoglobulin polypeptide, thereby rendering the retrovirus replication defective. Protocols for producing recombinant retroviruses and for infecting cells *in vitro* or *in vivo* with such viruses can be found in standard molecular biology laboratory manuals such as Current Protocols in Molecular Biology, Ausubel *et al.,* eds., 1989, Greene Publishing Associates. Examples of suitable retroviruses well known to those skilled in the art include but are not limited to pLJ, pZIP, pWE and pEM. Examples of suitable packaging virus lines for preparing both ecotropic and amphotropic retroviral systems include, but are not limited to, psiCrip, psiCre, psi2 and psiAm.

Furthermore, it is possible to limit the infection spectrum of retroviruses and consequently of retroviral-based vectors, by modifying the viral packaging proteins on the surface of the viral particle (see, for example, PCT publications WO 93/25234, WO 94/06920, and WO 94/11524, the contents of which is hereby incorporated by reference in their entireties). For instance, strategies for the modification of the infection spectrum of retroviral vectors include, but are not limited to, coupling antibodies specific for cell surface antigens to the viral env protein (Roux *et al.,* 1989, *Proc. Natl. Acad. Sci.,* 1989, 86: 9079-9083; Julan *et al.,* 1992, *Virol.* 73: 3251-3255; and Goud *et al.,* 1983, *Virology* 163: 251-254); or coupling cell surface ligands to the viral env proteins (*Neda et al.,* 1991, J. Biol. Chem. 266: 14143-14146)(the contents of which are incorporated herein by reference in their entireties). Coupling can be in the form of the chemical cross-linking with a protein or other moiety (for example, chemical coupling using lactose to convert the env protein to an a sialoglycoprotein), as well as by generating fusion proteins (single-chain antibody/env fusion proteins, for example). This technique, while useful to limit or otherwise direct the infection to certain tissue types, can also be used to convert an ecotropic vector into an amphotropic vector. Moreover, use of retroviral gene delivery can be further enhanced by the use of tissue- or cell-specific transcriptional regulatory sequences that control expression of the nucleic acid encoding an immunoglobulin polypeptide of the retroviral vector.

Another viral gene delivery system useful in the present invention utilizes adenovirus-derived vectors. The genome of an adenovirus can be manipulated such that it encodes a gene product of interest, but is inactivated in terms of its ability to replicate in a normal lytic viral life cycle (see, for example, Berkner *et al.,* 1988, BioTechniques 6: 616; Rosenfeld *et al.,* 1991, *Science* 252: 43 1434; and Rosenfeld *et al.,* 1992, *Cell* 68: 143-155, incorporated herein by reference in their entireties. Suitable adenoviral vectors derived from the adenovirus strain Ad type 5 dl324 or other strains of adenovirus (e.g., Ad2, Ad3, Ad7 etc.) are well known to those skilled in the art. The virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Most replication-defective adenoviral vectors currently in use and therefore favored by the present invention are deleted for all or parts of the viral E1 and E3 genes but retain as much as 80% of the adenoviral genetic material (see, for example, Jones et al., 1979, *Cell* 16: 683; Berkner et al., *supra;* and Graham et al. in Methods in Molecular Biology, E. J. Murray, *ed*., 1991, vol. 7, pp. 109-127 (Humana, Clifton, N.J.), which are incorporated herein by reference in their entireties. Expression of the inserted nucleic acid encoding an immunoglobulin polypeptide can be under control of, for example, the ElA promoter, the major late promoter (MLP) and associated leader sequences, the E3 promoter, exogenously added promoter sequences, and the like.

Yet another viral vector system useful for delivery of, for example, the subject nucleic acid encoding an immunoglobulin polypeptide, is the adeno-associated virus (AAV). Vectors containing as little as 300 base pairs of AAV can be packaged and can integrate. Space for heterologous DNA is limited to about 4.5 kb. An AAV vector such as that described in Tratschin *et al.,* 1985, *Mol. Cell. Biol.* 5: 3251-3260 can be used to introduce DNA into cells. A variety of nucleic acids have been introduced into different cell types using AAV vectors (see, for example, Hermonat *et al., Proc. Natl. Acad. Sci.,* 1984, 81: 6466-6470; Tratschin *et al.,* 1985, *Mol. Cell. Biol.* 4: 2072-2081 (1985); Wondisford *et al.,* 1988, *Mol. Endocrinol* 2: 32-39; Tratschin *et al.,* 1984, *J. Virol* 51: 611-619; and Flotte *et al.,* 1993, *J. Biol. Chem.* 268: 3781-3790), incorporated herein by reference in their entireties.

Other viral vector systems that may have application in the methods according to the present invention have been derived from, but are not limited to, herpes virus, *vaccinia* virus, avian leucosis virus, and several RNA viruses. For example, herpes virus vector variants may provide a unique strategy for persistence of a gene of interest expressed in cells of the central nervous system.

### Non-viral expression vectors

Most non-viral methods of gene transfer rely on the usual mechanisms employed by eukaryotic cells for the uptake and intracellular transport of macromolecules. In alternate embodiments, non-viral gene delivery systems of the present invention rely on endocytosis for the uptake of the subject nucleic acid encoding an immunoglobulin polypeptide by the targeted cell. Exemplary gene delivery systems of this type include liposomal derived systems, poly-lysine conjugates, and artificial viral envelopes.

In a representative embodiment of the present invention, a nucleic acid encoding an immunoglobulin polypeptide can be entrapped in liposomes bearing positive charges on their surface (e.g., lipofectins) and (optionally) which are tagged with antibodies against cell surface antigens of the target tissue (Mizuno *et al., NO Shinkei Geka,* 1992, 20: 547-551; PCT publication WO 91/06309; Japanese patent application 1047381; and European patent publication EP-A-43075, incorporated herein by reference in their entireties).

In similar fashion, the gene delivery system comprises an antibody or cell surface ligand that is cross-linked with a gene binding agent such as polylysine (see, for example, PCT publications VVO 93/04701, WO 92/22635, WO 92/20316, WO 92/19749, and WO 92/06180, incorporated herein by reference in their entireties). It will also be appreciated that effective delivery of the subject nucleic acid constructs via receptor-mediated endocytosis can be improved using agents which enhance escape of a gene from the endosomal structures. For instance, whole adenovirus or fusogenic peptides of the influenza HA gene product can be used as part of the delivery system to induce efficient disruption of DNA-containing endosomes (Mulligan *et al.,* 1993, *Science* 260: 926; Wagner *et al.,* 1992, *PNAS* 89: 7934; and Christiano *et al.,* 1993, *PNAS* 90: 2122, incorporated herein by reference in their entireties).

### Transgenic birds

Another aspect of the present invention concerns transgenic birds including, but not limited to, chickens having at least one transgene and that preferably (though optionally) express the subject nucleic acid encoding an immunoglobulin polypeptide in one or more cells in the animal as, for example, the oviduct cells of the chicken. In embodiments of the present invention, therefore, the expression of the transgene is restricted to specific subsets of cells, tissues, or developmental stages utilizing, for example, cis-acting sequences that control expression in the desired pattern. Toward this end, tissue-specific regulatory sequences, tissue-specific promoters, and conditional regulatory sequences can be used to control expression of the transgene in certain spatial patterns. Moreover, temporal patterns of expression can be provided by, for example, conditional recombination systems, prokaryotic transcriptional regulatory sequences, and the like.

Conditional transgenes can be provided using prokaryotic promoter sequences that require prokaryotic proteins to be simultaneous expressed to facilitate expression of the transgene. Operators present in prokaryotic cells have been extensively characterized *in vivo* and *in vitro* and can be readily manipulated to place them in any position upstream from, or within, a gene by standard techniques. Such operators comprise promoter regions and regions that specifically bind proteins such as activators and repressors. One example is the operator region of the *lexA* gene of *E. coli* to which the LexA polypeptide binds. Other exemplary prokaryotic regulatory sequences and the corresponding trans-activating prokaryotic proteins are disclosed by Brent and Ptashne in U.S. Patent No. 4,833,080. Transgenic animals can be created which harbor the subject transgene under transcriptional control of a prokaryotic sequence that is not appreciably activated by eukaryotic proteins. Breeding of this transgenic animal with another animal that is transgenic for the corresponding prokaryotic transactivator can permit activation of the nucleic acid encoding an immunoglobulin polypeptide. Moreover, expression of a conditional transgene can be induced by gene therapy-like methods (such as described above) wherein a gene encoding the trans-activating protein, e.g. a recombinase or a prokaryotic protein, is delivered to the tissue and caused to be expressed, such as in a cell-type specific manner.

Additionally, inducible promoters can be employed according to the present invention. Examples of inducible promoters include, but are not limited to, the tet operator and the metallothionein promoter which can be induced by treatment with tetracycline and zinc ions, respectively (Gossen *et al.,* 1992, *PNAS* 89: 5547-5551 and Walden *et al.,* 1987, *Gene* 61: 317-327, incorporated herein by reference in their entireties).

### Cloned-, Transgenic-, and Knock-in Animals and Their Eggs

Methods of producing a transgenic animal, as contemplated by the present invention, include introducing a transgene to an animal using: a viral or a non-viral vector; sperm-mediated gene transfer; restriction enzyme-mediated integration; nuclear transfer, including nuclear transfer using two-photon visualization and, optionally, laser-mediated ablation; ovum transfer; and the like. In the case of an avian, a heterologous immunoglobulin polypeptide or polypeptides encoded by the transgenic nucleic acid may be secreted into the oviduct lumen of the mature animal and deposited as a constituent component of the egg white into eggs laid by the animal. It is also contemplated to be within the scope of the present invention for the heterologous immunoglobulin polypeptides to be produced in the egg yolk or in the serum of a transgenic avian. In one embodiment contemplated by the method of the present invention, a leaky promoter, such as the CMV promoter, may be operably linked to a transgene resulting in expression of the transgene in many, if not all, of the tissues of the transgenic avian, resulting in production of immunoglobulin polypeptides in the serum. Transgenic avians produced by the present invention will be able to lay eggs containing one or more desired heterologous protein(s), including for example, an immunoglobulin light or heavy chain, an antibody or variant thereof, and the like.

A transgene may be introduced into the ovum of an animal, according to the present invention, by nuclear transfer via two-photon visualization and ablation, wherein the nuclear donor contains a desired heterologous DNA sequence in its genome, such as a DNA encoding at least one immunoglobulin polypeptide. One of ordinary skill in the art will be able to readily adapt conventional methods to insert the desired transgene into the genome of the nuclear donor prior to injection of the nuclear donor into the recipient cytoplast, or prior to fusion of the nuclear donor cell with the recipient cell. For example, a vector that contains one or more transgene(s) encoding at least one polypeptide chain of an antibody, may be delivered into the nuclear donor cell through the use of a delivery vehicle. The transgene is then transferred along with the nuclear donor into the recipient ovum. Following zygote reconstruction, the ovum is transferred into the reproductive tract of a recipient hen. In one embodiment of the present invention, the ovum is transferred into the infundibulum of the recipient hen. After reconstruction, the embryo containing the transgene develops inside the recipient hen and travels through the oviduct thereof, where it is encapsulated by natural egg white proteins and a natural egg shell. The egg is laid and can be incubated and hatched to produce a transgenic chick. The resulting transgenic chick will carry one or more desired transgene(s) in its germ line. Following maturation, the transgenic avian may lay eggs that contain one or more desired heterologous protein(s) that can be easily harvested.

In another embodiment of the present invention, a nuclear donor cell is transfected with a vector construct that contains a transgene encoding at least one polypeptide chain of an antibody or a variant or truncated form thereof. Methods for transfection of somatic cell nuclei are well known in the art and include, by way of example, the use of retroviral vectors, retrotransposons, adenoviruses, adeno-associated viruses, naked DNA, lipid-mediated transfection, electroporation, direct injection into the nucleus, and the like. Such techniques, particularly as applied to avians, are disclosed in Bosselman (U.S. Patent No. 5,162,215), Etches (PCT Publication No. WO 99/10505), Hodgson (U.S. Patent No. 6,027,722), Hughes (U.S. Patent No. 4,997,763), Ivarie *et al.* (PCT Publication No. WO 99/19472), MacArthur (PCT Publication No. WO 97/47739), Perry (U.S. Patent No. 5,011,780), Petitte (U.S. Patent Nos. 5,340,740 and 5,656,749), and Simkiss (PCT Publication No. WO 90/11355), the disclosures of which are incorporated by reference herein in their entireties.

### Nuclear Transfer and TPLSM

Nuclear transfer allows the cloning of animal species, wherein individual steps are common to the procedures of embryonic, fetal, and adult cell cloning. These steps include, but are not limited to: a). preparation of a cytoplast, b). donor cell nucleus (nuclear donor) isolation and c). transfer of the donor nucleus to the cytoplast to produce a reconstructed embryo. Optionally, additional steps include d). culture of the reconstructed embryo and e). transfer of the reconstructed embryo to a synchronized host animal.

In one embodiment of the present invention, the nuclear transfer approach used in animals employs nuclear visualization using a two-photon microscope. The animal used for nuclear transfer may be an avian including, but not limited to, chickens, ducks, turkeys, quails, pheasants and ratites. In this method, a fertilized or unfertilized egg is removed from an animal and manipulated *in vitro,* wherein the genetic material of the egg is visualized and removed or ablated and the ablated nucleus replaced with a donor nucleus. Optionally, the donor nucleus may be genetically modified with, for example, a transgene encoding an immunoglobulin polypeptide. Two-photon laser scanning microscopy (TPLSM) may be used to visualize the nuclear structures. Following visualization, the nucleus in the recipient cell, such as a fertilized or unfertilized egg, is removed or ablated, optionally using visualization by TPLSM.

TPLSM is based on two-photon excited fluorescence in which two photons collide simultaneously with a fluorescent molecule. Their combined energy is absorbed by the fluorophore, inducing fluorescent emission that is detected by a photomultiplier tube and converted into a digital image. See Squirrell *et al.,* 1999, *Nature Biotechnol.* 17: 763-7 and Piston *et al.,* 1999, *Trends Cell Biol.* 9: 66-9, incorporated herein by reference in their entireties. TPLSM generates images of living, optically-dense structures for prolonged periods of time while not affecting their viability. TPLSM utilizes biologically innocuous pulsed near-infrared light, usually at a wavelength of about 700 nm to about 1000 nm, which is able to penetrate deep into light-scattering specimens. TPLSM may employ different lasers, such as a mode-locked laser where the wavelength is fixed, or a tunable laser that can be tuned to wavelengths between about 700 nm and about 1000 nm, depending upon the range of emission of the dye used. For example, with the use of DAPI and Hoescht 33342 dyes, a wavelength of 720-770 nm is preferred. New fluorophores are being produced with different ranges of emission and the invention is not limited to the presently available dyes and their respective emission ranges.

Furthermore, lasers used in TPLSM can be grouped into femtosecond and picosecond lasers. These lasers are distinguished by their pulse duration. A femtosecond laser is presently preferred, since it is particularly suitable for visualization without harming the specimen.

TPLSM produces noninvasive, three-dimensional, real-time images of the optically dense avian egg. In contrast to mammalian cells, visualization of the metaphase plate or pronucleus in the avian egg during nuclear transfer has been hampered or prevented by the large, opaque avian yolk. Two-photon imaging with femtosecond lasers operating in the near infrared, however, allows visualization of avian nuclear structures without damaging cellular constituents. In one embodiment of the present invention, specimens may be incubated or injected with DNA-specific dyes, such as DAPI (4', 6'-diamidino-2-phenylindole hydrochloride) or Hoescht 33342 (bis-benzimide) prior to TPLSM visualization, followed by removal of the albumen capsule and placement of the ovum in a dish with the germinal disk facing the top. Remnants of the albumen capsule may then be removed from the top of the germinal disk.

An aqueous solution such as, for example, phosphate-buffered saline (PBS) may be added to the dish or directly onto the ovum to prevent drying of the ovum. A cloning cylinder may then be placed around the germinal disk and DAPI in PBS added to the cylinder. Alternatively, a DAPI-PBS solution may be injected into the germinal disk with a glass pipette, whereupon the dye enters the nuclear structures. For dye injection, removal of the albumen capsule is not necessary whereas injection of nuclei into the disk is facilitated in the absence of the capsule.

Images of the inside of the early avian embryo can be generated through the use of TPLSM. Visualization may be performed after about 10 to 15 minutes of incubation with the dye or about 10 minutes after dye injection. During visualization, the germinal disk is placed under the microscope objective and the pronuclear structures are searched within the central area of the disk using relatively low laser powers of about 3-6 milliwatts. Once the structures are found, they may be ablated by using higher laser power or mechanically removed guided by TPLSM visualization.

Nuclear transfer techniques require the destruction or removal (enucleation) of the pronucleus before a nuclear donor can be introduced into the oocyte cytoplast. Two-photon laser-mediated ablation of nuclear structures provides an alternative to microsurgery to visualize the pronucleus lying about 25µm beneath the ovum's vitelline membrane within the germinal disk. Higher laser powers than those used for imaging can be used for enucleation, with minimal collateral damage to the cell. The wavelength for ablation generally ranges from about 700 nm to about 1000 nm, at about 30 to about 70 milliwatts. TPLSM and two-photon laser-mediated ablation are more efficient than alternative methods known in the art because they are less operator-dependent and less invasive, resulting in improved viability of the recipient cell.

A nucleus from a cultured somatic cell (nuclear donor) may then be injected into the enucleated recipient cytoplast. In one embodiment of the present invention, the nuclear donor is injected using a micromanipulation unit comprising a microinjector and a micromanipulator. The donor nucleus is introduced into the germinal disk though guided injection using episcopic illumination (i.e., light coming through the microscope objective onto the sample). Alternatively, a donor cell may be fused to the recipient cell using methods well known in the art, e.g., by means of fusion-promoting chemicals, such as polyethylene glycol; by inactivated viruses, such as Sendai virus; or through electrical stimulation. The reconstructed zygote may then be surgically transferred to the oviduct of a recipient hen to produce a hard shell egg. Alternatively, the reconstructed embryo may be cultured in vitro to permit screening the embryo for proper development prior to transfer into a recipient. For example, one embodiment of the present invention contemplates culturing the reconstructed embryo for about 24 hours prior to screening and subsequent surgical transfer into a recipient hen.

The egg can be harvested after laying and before hatching of a chick, or further incubated to generate a cloned chick, optionally a cloned chick that has been genetically modified. The cloned chick may carry a transgene in all, most, or a few of its cells. After maturation, the transgenic chick may lay eggs that contain one or more desired, heterologous protein(s) including an antibody capable of selectively binding to an antigen, or an immunoglobulin polypeptide that may be isolated and associated with another isolated immunoglobulin polypeptide, thereby forming an antibody capable of selectively binding to an antigen. The cloned chick may also be a knock-in chick expressing an alternative phenotype or capable of laying eggs having an heterologous protein therein. The reconstructed egg may also be cultured to term using an ovo method of culture. For example, the *ex ovo* culture method described by Perry *et al. (supra)* is contemplated to be within the scope of the method of the present invention.

### Ovum Transfer

Another aspect of the present invention provides for a method of producing a cloned animal comprising nuclear transfer in combination with ovum transfer. Two-photon visualization and ablation may be used to perform nuclear transfer, as described above. Accordingly, the replacement of the recipient cell's nucleus with the donor cell's nucleus results in a reconstructed zygote. In one embodiment, pronuclear stage eggs are used as recipient cytoplasts already activated by fertilization. Alternatively, unactivated metaphase II eggs may serve as recipient cytoplast and activation induced after renucleation. The ovum may be cultured via ovum transfer, wherein the ovum containing the reconstructed zygote is transferred to a recipient hen. The ovum is surgically transferred into the oviduct of the recipient hen shortly after oviposition. This is accomplished according to normal husbandry procedures (oviposition, incubation, and hatching; see Tanaka *et al., .supra).*

Alternatively, the ovum may be cultured to stage X prior to transfer into a recipient hen. More specifically, reconstructed stage I embryos are cultured for 24-48 hours to stage X. This allows for developmental screening of the reconstructed embryo prior to transfer. Stage I embryos are enclosed within a thick albumen capsule. In this novel procedure, the albumen capsule is removed, after which the nuclear donor is injected into the germinal disk. Subsequently, the capsule and germinal disk are recombined by placing the thick capsule in contact with the germinal disk on top of the yolk. Embryos develop to stage X at similar rates as those cultured with their capsules intact. At stage X, the embryo is transferred to the oviduct of a recipient hen.

Once transferred, the embryo develops inside the recipient hen and travels through the oviduct of the hen where it is encapsulated by natural egg white proteins and a natural egg shell. The egg, containing endogenous yolk and an embryo from another hen, is laid and can then be incubated and hatched like a normal chick. The resulting chick may carry a transgene in all or most of its cells. In one embodiment, the transgene is at least in the oviduct cells of the recipient chick. Following maturation, the cloned avian may express a desired phenotype or may be able to lay eggs that contain one or more desired, heterologous protein(s).

### Sperm-mediated integration of heterologous transgenes

Detailed descriptions of methods of sperm-mediated transfer of nucleic acid suitable for use in the present invention are described *inter alia* in PCT Publication WO 00/697257; WO 99/42569; WO 00/09674; WO 01/19183; and in U.S. Patent No. 5,804,191 to Scofield, incorporated herein by reference in their entireties. One method of incorporating heterologous genetic material into the genome of an avian delivers a nucleic acid using known gene delivery systems to male germ cells *in situ* in the testis of the male avian (e.g., by *in vivo* transfection or transduction). Alternatively, an *in vitro* method of incorporating heterologous genetic material into the genome of an avian involves isolating male germ cells *ex corpora,* delivering a polynucleotide thereto, and then returning the transfected cells to the testes of a recipient male bird.

### In vivo method

One *in vivo* method contemplated for use in the present invention employs injection of the gene delivery mixture, preferably into the seminiferous tubules, or into the pete testis, and most preferably into the vas efferens or vasa efferentia, using, for example, a micropipette and a picopump delivering a precise measured volume under controlled amounts of pressure. A small amount of a suitable, non-toxic dye can be added to the gene delivery mixture (fluid) to confirm delivery and dissemination to the seminiferous tubules of the testis. The genetically modified germ cells differentiate in their own milieu. Progeny animals exhibiting the nucleic acid's integration into its germ cells (transgenic animals) are selected. The selected progeny can then be mated, or their sperm utilized for insemination or *in vitro* fertilization to produce further generations of transgenic progeny.

### In vitro method

In an alternative method, male germ cells are obtained or collected from the donor male bird by any means known in the art such as, for example, transection of the testes. The germ cells are then exposed to a gene delivery mixture, preferably within several hours, or cryopreserved for later use. When the male germ cells are obtained from the donor vertebrate by transection of the testes, the cells can be incubated in an enzyme mixture known for gently breaking up the tissue matrix and releasing undamaged cells such as, for example, pancreatic trypsin, collagenase type I, pancreatic DNAse type I, as well as bovine serum albumin and a modified DMEM medium. After washing the cells, they can be placed in an incubation medium such as DMEM and the like, and plated on a culture dish for genetic modification by exposure to a gene delivery mixture.

Whether employed in the *in vivo* method or *in vitro* method, the gene delivery mixture, once in contact with the male germ cells, facilitates the uptake and transport of heterologous genetic material into the appropriate cell location for integration into the genome and expression. A number of known gene delivery methods can be used for the uptake of nucleic acid sequences into the cell. Such methods include, but are not limited to, viral vectors, liposomes, electroporation, Restriction Enzyme Mediated Integration (REMI) (discussed below) and the like. In both the *in vivo* or *in vitro* method, a gene delivery mixture typically comprises a polynucleotide encoding the desired trait or product (for example, immunoglobulin polypeptides) and a suitable promoter sequence such as, for example, a tissue-specific promoter, an IRES, and the like and, optionally, agents that increase the uptake of or comprise the polynucleotide sequence, such as liposomes, retroviral vectors, adenoviral vectors, adenovirus enhanced gene delivery systems, and the like or combinations thereof. A reporter construct, including a genetic selection marker such as the gene encoding for Green Fluorescent Protein, can further be added to the gene delivery mixture. Targeting molecules, such as the c-kit ligand, can be added to the gene delivery mixture to enhance the transfer of genetic material into the male germ cell. An immunosuppressing agent, such as cyclosporin or a corticosteroid, may also be added to the gene delivery mixture as known in the art.

Any of a number of commercially available gene delivery mixtures can be used, to which the polynucleotide encoding a desired trait or product is further admixed. The final gene delivery mixture comprising the polynucleotide can then be admixed with the cells and allowed to interact for a period of between about 2 hours to about 16 hours, at a temperature of between about 33°C to about 37°C. After this period, the cells are preferably placed at a lower temperature, of about 33 °C to about 34°C, for about 4 hours to about 20 hours, preferably about 16 to 18 hrs.

Isolating and/or selecting genetically transgenic germ cells (and transgenic somatic cells, and transgenic vertebrates) is by any suitable means, such as but not limited to, physiological and/or morphological phenotypes of interest using any suitable means, such as biochemical, enzymatic, immunochemical, histologic, electrophysiologic, biometric or like methods, and analysis of cellular nucleic acids, as, for example, the presence or absence of specific DNAs or RNAs of interest using conventional molecular biological techniques, including hybridization analysis, nucleic acid amplification including, but not limited to, polymerase chain reaction, transcription-mediated amplification, reverse transcriptase-mediated ligase chain reaction, and/or electrophoretic technologies.

One method contemplated by the present invention for isolating or selecting male germ cell populations comprises obtaining specific male germ cell populations, such as spermatogonia, from a mixed population of testicular cells by extrusion of the cells from the seminiferous tubules and enzyme digestion. The spermatogonia, or other male germ cell populations, can be isolated from a mixed cell population by known methods such as the utilization of a promoter sequence that is specifically or selectively active in cycling male germ line stem cell populations. Suitable promoters include B-Myb or a specific promoter, such as the c-kit promoter region, c-raf promoter, ATM (ataxia-telangiectasia) promoter, vasa promoter, RBM (ribosome binding motif) promoter, DAZ (deleted in azoospermia) promoter, XRCC- 1 promoter, HSP 90 (heat shock gene) promoter, cyclin Al promoter, or FRMI (from Fragile X site) promoter and the like. A selected promoter may be linked to a reporter construct including, for example, a construct comprising a gene encoding Green Fluorescent Protein (or EGFP), Yellow Fluorescent Protein, Blue Fluorescent Protein, a phycobiliprotein such as phycoerythrin or phycocyanin, or any other protein which fluoresces under a suitable wave-length of light, or encoding a light-emitting protein, such as luciferase or apoaequorin. The unique promoter sequences drive the expression of the reporter construct only during specific stages of male germ cell development (e.g., Mailer *et al.,* 1999, *J Biol. Chem.* 276(16): 11220-28; Schrans-Stassen *et al.,* 1999, *Endocrinology* 140: 5894-5900, incorporated herein by reference in their entireties). In the case of a fluorescent reporter construct, the cells can be sorted with the aid of, for example, a FACS set at the appropriate wavelength(s), or they can be selected by chemical methods.

Male germ cells that have the DNA modified in the desired manner are isolated or selected, and transferred to the testis of a suitable recipient animal. Further selection can be attempted after biopsy of one or both of the recipient male's testes, or after examination of the animal's ejaculate amplified by the polymerase chain reaction to confirm that the desired nucleic acid sequence had been incorporated.

The genetically modified germ cells isolated or selected as described above are preferably transferred to a testis of a recipient male avian, e.g., a chicken, that can be, but need not be, the same donor animal. Before transferring the genetically modified male germ cells to the recipient animal, the testes of the recipient can be depopulated of endogenous germ cells, thereby facilitating the colonization of the recipient testis by the genetically modified germ cells, by any suitable means, including by gamma irradiation, by chemical treatment, by means of infectious agents such as viruses, or by autoimmune depletion or by combinations thereof. In one embodiment of the present invention, the testis is depopulated using a treatment combining administration of an alkylating agent with gamma irradiation.

Any method known in the art for depopulating the testis may be used in the present invention provided that the basic rigid architecture of the gonad should not be destroyed, nor significantly damaged by the treatment. For example, disruption of the seminiferous tubules may lead to impaired transport of testicular sperm and result in infertility. Nor should the treatment chosen irreversibly damage the sertoli cells, as they provide a base for development of the germ cells during maturation, and for preventing the host immune defense system from destroying grafted foreign spermatogonia.

In on embodiment of the present invention, a cytotoxic alkylating agent, such as but not limited to, bisulfan (1,4-butanediol dimethanesulphonate), chlorambucil, cyclophosphamide, melphalan, ethyl ethanesulfonic acid, or the like is combined with gamma irradiation, to be administered in either sequence. The dose of the alkylating agent and the dose of gamma radiation are in an amount sufficient to substantially depopulate the testis. The alkylating agent can be administered by any pharmaceutically acceptable delivery system including, but not limited to, intraperitoneal, intravenous, or intramuscular injection, intravenous drip, implant, transdermal or transmucosal delivery systems.

The isolated or selected genetically modified germ cells can be transferred into the recipient testis by direct injection using a suitable micropipette. Support cells, such as Leydig or Sertoli cells, that can be unmodified or genetically modified can be transferred to a recipient testis along with the modified germ cells.

A union of male and female gametes to form a transgenic zygote is brought about by copulation of the male and female vertebrates of the same species, or by *in vitro* or *in vivo* artificial means. If artificial means are chosen, then incorporating into the genome a genetic selection marker that is expressed in male germ cells is particularly useful.

Suitable artificial means include, but are not limited to, artificial insemination, *in vitro* fertilization (IVF) and/or other artificial reproductive technologies, such as intracytoplasmic sperm injection (ICSI), subzonal insemination (SUZI), or partial zona dissection (PZD). Also other methods, such as cloning and embryo transfer, cloning and embryo splitting, and the like, can be employed in the method of the present invention.

The transgenic vertebrate progeny can, in turn, be bred by natural mating, artificial insemination, by *in vitro* fertilization (IVF) and/or other artificial reproductive technologies. For example, intracytoplasmic sperm injection (ICSI) and chicken intracytoplasmic sperm injection (CHICSI^{™}), subzonal insemination (SUZI), or partial zona dissection (PZD) can be used to obtain further generations of transgenic progeny. Although the genetic material is originally inserted solely into the germ cells of a parent animal, it will ultimately be present in the germ cells of future progeny and subsequent generations thereof. In addition, the genetic material may also be present in cells of the progeny other than germ cells, i.e., somatic cells.

### Restriction Enzyme-Mediated Integration (REMI)

The REMI method for stably integrating heterologous DNA into the genomic DNA of a recipient cell, as described by Shemesh *et al.* in PCT Publication WO 99/42569 and incorporated herein by reference in its entirety, comprises in part an adaptation of the REMI techniques disclosed by Schiest and Petes (1991, *PNAS U.S.A.* 88: 7585-7589) and Kuspa and Loomis (1992, *PNAS U.S.A.* 89: 8803-8807), both incorporated herein by reference in their entireties.

The REMI method is suitable for introducing heterologous DNA into the genome nucleic acid of sperm and sperm precursor cells, or ovum, embryonic cell, or somatic cell of an animal, preferably an avian, more preferably a chicken.

The heterologous nucleic acid to be integrated into, for example, the sperm nuclear DNA is converted to a linear double stranded DNA possessing single-stranded cohesive ends by contacting the heterologous DNA with a type II restriction enzyme that upon scission, generates such ends. The nucleic acid to be cut can be a circular nucleic acid, such as in a plasmid or a viral vector, or a linear nucleic acid that possesses at least one recognition and cutting site outside of the genes or regulatory regions critical to the desired post-integration function of the nucleic acid, and no recognition and cutting sites within the critical regions.

Alternatively the heterologous DNA to be integrated into the sperm nuclear DNA can be prepared by chemically and/or enzymatically adding cohesive ends to a linear DNA (*see,* for example Sambrook *et al., Molecular Cloning: A Laboratory Manual,* 2nd ed., Cold Spring Harbor Laboratory, Cold Spring Harbor, New York (1989) and incorporated herein by reference in its entirety). The added cohesive ends must be able to hybridize to the cohesive ends characteristic of a nucleic acid cleaved by a type II restriction endonuclease. Alternatively, the cohesive ends can be added by combining the methods based on type II restriction enzyme cutting and chemical and/or enzymatic addition.

According to the present invention, a heterologous nucleic acid, encoding at least one immunoglobulin polypeptide, and the appropriate restriction enzyme can be introduced into sperm cells together or sequentially by way of, for example, electroporation or lipofection. However, the present invention contemplates that any technique capable of transferring heterologous material into sperm could be used so long as the technique preserves enough of the sperm's fertilization functions, such that the resultant sperm will be able to fertilize the appropriate oocytes. It is understood that the heterologous nucleic acid may be integrated into the genome of a recipient cell, such as a spermatogonial cell or a spermatogonial precursor cell, for subsequent transfer to an embryo or to the testicular material of the recipient male animal, preferably a chicken. It is further understood that the heterologous nucleic acid may not be integrated into the genome of the recipient cell (e.g., carried episomally).

The combination of REMI, as described in the present application, combined with a relatively benign method of transferring heterologous material into a cell may result in heterologous nucleic acid being stably integrated into genomic DNA of a high fraction of the treated sperm, while not diminishing to any great extent, the viability of the sperm or their ability to fertilize oocytes. Examples of suitable methods for the introduction of the genetically modified sperm, spermatogonial cells, or precuror spermatogonial cells into a recipient avian, preferably a chicken, are as described above.

It is contemplated to be within the scope of the present invention for nucleic acids encoding immunoglobulin polypeptides, and the immunoglobulin polypeptides and antibody molecules formed therefrom, to be derived from any suitable species including, for example, a human, a mouse, a rat, a rabbit, a goat, a sheep, a cow, a horse or a bird. The antibodies, or nucleic acids encoding thereof, may be monoclonal antibodies. It is further within the scope of the present invention for the immunoglobulin polypeptides and antibodies derived therefrom to be modified, for example, by exchanging regions within the polypeptides from one animal species for equivalent regions from another species. It is further understood that an immunoglobulin polypeptide from one animal species may be combined with an immunoglobulin polypeptide from another animal species.

One aspect of the present invention is a method for the production of an antibody by an avian cell comprising the step of culturing an avian cell transformed with a first expression vector and, optionally, a second expression vector; the expression vectors each having a transcription unit comprising a nucleotide sequence encoding at least one immunoglobulin polypeptide, a transcription promoter, and a transcriptional terminator operatively linked to the nucleotide sequence encoding at least one immunoglobulin polypeptide and wherein the cultured avian cell produces an antibody selectively binding an antigen. Illustrative examples of this aspect of the present invention are presented herein in Examples 1 and 2 below.

In one embodiment of the method of the present invention, the avian cell is transformed with at least one expression vector comprising a transcription unit encoding at least one immunoglobulin polypeptide selected from an immunoglobulin heavy chain variable region, an immunoglobulin heavy chain comprising a variable region and a constant region, an immunoglobulin light chain variable region, an immunoglobulin light chain comprising a variable region and a constant region, and a single-chain antibody comprising two linked immunoglobulin variable regions.

In another embodiment of the method of the present invention, the avian cell is transformed with an expression vector comprising a transcription unit encoding a first immunoglobulin polypeptide and a second immunoglobulin polypeptide, the first and second immunoglobulin polypeptides being selected from an immunoglobulin heavy chain variable region, an immunoglobulin heavy chain comprising a variable region and a constant region, an immunoglobulin light chain variable region, an immunoglobulin light chain comprising a variable region and a constant region, and further comprising an internal ribosome entry site (IRES) operatively linked to the second immunoglobulin polypeptide. The IRES will allow for translation of the second immunoglobulin polypeptide as an individual polypeptide.

In still another embodiment of the method of the present invention, the individual immunoglobulin polypeptide may have peptide regions that are suitable for the isolation of the immunoglobulin polypeptide as, for example, a polyhistidine peptide for binding to a Ni⁺-containing column.

In the embodiments of the present invention contemplated within the scope of the present invention, the avian cell may be a cell from a chicken, a turkey, a duck, a goose, a quail, a pheasant, a ratite, an ornamental bird or a feral bird, most preferably from a chicken. The avian cell may be selected from, but is not limited to, a somatic cell such as a fibroblast, an oviduct cell, an embryonic cell and the like or, alternately, be a germ-line ovum or a testicular cell, preferably from an embryonic cell, or an oviduct cell.

It is contemplated to be within the scope of the present invention for the expression vectors to include, but not be limited to, viral vectors, plasmid vectors, linear nucleic acid vectors, or the like or a combination thereof.

The expression vector may be any suitable viral vector as, for example, an avian leucosis virus vector, an adenoviral vector, a transferrin-polylysine enhanced adenoviral vector, a human immunodeficiency virus vector, a lentiviral vector, a Moloney murine leukemia virus-derived vector, and the like or, alternately, virus-derived DNAs that facilitate polynucleotide uptake by, and release into, the cytoplasm of germs cells.

Transcriptional promoters of an expression vector of the present invention may be a constitutively active promoter, such as the cytomegaloviral promoter, or a tissue-specific promoter. For example, one embodiment of the present invention contemplates the use of a tissue-specific promoter operable in oviduct cells of an avian species including, but not limited to, the promoters of the genes encoding ovalbumin, lysozyme, ovomucoid, ovotransferrin (conalbumin), ovomucin and the like. Optionally, the transcriptional promoter of an expression vector may be a regulatable promoter.

The transcriptional terminator of at least one expression vector may further comprise a region encoding a transcriptional terminator, such as a bovine growth hormone transcriptional terminator.

In the various embodiments of this aspect of the present invention, an immunoglobulin polypeptide encoded by the transcriptional unit of at least one expression vector may be an immunoglobulin heavy chain polypeptide comprising a variable region or a variant thereof, and may further comprise a D region, a J region, a C region, or a combination thereof. An immunoglobulin polypeptide encoded by the transcriptional unit of an expression vector may also be an immunoglobulin light chain polypeptide comprising a variable region or a variant thereof, and may further comprise a J region and a C region. It is also contemplated to be within the scope of the present invention for the immunoglobulin regions to be derived from the same animal species, or a mixture of species including, but not only, human, mouse, rat, rabbit and chicken.

In other embodiments of the present invention, the immunoglobulin polypeptide encoded by the transcriptional unit of at least one expression vector comprises an immunoglobulin heavy chain variable region, an immunoglobulin light chain variable region, and a linker peptide thereby forming a single-chain antibody capable of selectively binding an antigen.

Another aspect of the present invention provides a method for the production in an avian of an heterologous protein capable of forming an antibody suitable for selectively binding an antigen comprising the step of producing a transgenic avian incorporating at least one transgene, wherein the transgene encodes at least one heterologous polypeptide selected from an immunoglobulin heavy chain variable region, an immunoglobulin heavy chain comprising a variable region and a constant region, an immunoglobulin light chain variable region, an immunoglobulin light chain comprising a variable region and a constant region, and a single-chain antibody comprising two peptide-linked immunoglobulin variable regions. Additional steps of the method of the present invention include depositing the heterologous polypeptide in the white of the developing eggs of the avian, harvesting the hard shell avian eggs thus produced and are harvested, and isolating the heterologous polypeptide capable of forming an antibody from the harvested egg. It is also understood that the heterologous polypeptides may also be expressed under the transcriptional control of promoters that allow for release of the polypeptides into the serum of the transgenic animal. An exemplary promoter for non-tissue specific production of a heterologous protein is the CMV promoter.

In one embodiment of this method of the present invention, the transgene comprises a transcription unit encoding a first and a second immunoglobulin polypeptide operatively linked to a transcription promoter, a transcription terminator and, optionally, an internal ribosome entry site (IRES)(see, for example, U.S. Patent No. 4,937,190 to Palmenberg *et al.,* the contents of which is incorporated herein by reference in its entirety).

In an embodiment of this method of the present invention, the isolated heterologous protein is an antibody capable of selectively binding to an antigen. In this embodiment, the antibody may be generated within the serum of an avian or within the white of the avian egg by combining at least one immunoglobulin heavy chain variable region and at least one immunoglobulin light chain variable region, preferably cross-linked by at least one cysteine bridge. The combination of the two variable regions will generate a binding site capable of binding an antigen.

It is, however, contemplated to be within the scope of the present invention for immunoglobulin heavy and light chains, or variants or derivatives thereof, to be expressed in separate transgenic avians, and therefore isolated from separate media including serum or eggs, each isolate comprising a single species of immunoglobulin polypeptide. The method may further comprise the step of combining a plurality of isolated heterologous immunoglobulin polypeptides, thereby producing an antibody capable of selectively binding to an antigen. In this embodiment, two individual transgenic avians may be generated wherein one transgenic produces serum or eggs having an immunoglobulin heavy chain variable region, or a polypeptide comprising such, expressed therein. A second transgenic animal, having a second transgene, produces serum or eggs having an immunoglobulin light chain variable region, or a polypeptide comprising such, expressed therein. The polypeptides may be isolated from their respective sera and eggs and combined *in vitro* to generate a binding site capable of binding an antigen.

In one embodiment of this method of the present invention, the transgenic avian having a transgene encoding at least one immunoglobulin polypeptide, for example a transgenic chicken, is produced by introducing a transgenic avian donor nucleus into a recipient cell to produce a reconstructed avian zygote, activating the reconstructed zygote, and allowing the reconstructed zygote to develop to term. The recipient cell may be an enucleated cell and may be visualized using a two photon laser scanning microscope.

In another embodiment of this aspect of the present invention, the transgenic avian may be produced by the sperm-mediated transfer of at least one transgene, wherein the at least one transgene encodes an immunoglobulin heavy chain variable region, an immunoglobulin heavy chain comprising a variable region and a constant region, an immunoglobulin light chain variable region, an immunoglobulin light chain comprising a variable region and a constant region, or an single-chain antibody comprising two linked immunoglobulin variable regions, and wherein the transgene is incorporated into the genome of a spermatozoal cell or a precursor thereof, so that a genetically modified male gamete is produced by the male avian. Breeding the male avian with a female of its species will generate a transgenic progeny carrying the at least one transgene in its genome.

In still another embodiment of this aspect of the present invention, the transgene is integrated into the genomic DNA of an avian sperm by an *in vivo* method comprising the steps of administering to an avian testis, preferably a chicken testis, a gene delivery mixture comprising a viral vector that comprises at least one polynucleotide encoding at least one heterologous immunoglobulin polypeptide; the heterologous polypeptide being selected, for example, from an immunoglobulin heavy chain variable region, an immunoglobulin heavy chain comprising a variable region and a constant region, an immunoglobulin light chain variable region, an immunoglobulin light chain comprising a variable region and a constant region, and a single-chain antibody comprising two linked immunoglobulin variable regions; the heterologous nucleotide being operatively linked to a transcriptional promoter sequence such that a transcriptional unit is formed under conditions effective to reach a spermatozoon cell or a precursor cell within the testis. The precursor cell may be selected from the group consisting of spermatogonial stem cells, type B spermatogonia, primary spermatocytes, preleptotene spermatocytes, leptotene spermatocytes, zygotene spermatocytes, pachytene spermatocytes, secondary spermatocytes, spermatids and the like. The embodiment may further comprise the steps of incorporating the polynucleotide encoding the at least one heterologous polypeptide into the genome of the spermatozoon cell or the precursor cell, so that a genetically modified male gamete is produced by the male avian, and breeding the male avian with a female of the same species such that a transgenic progeny is thereby produced that carries the polynucleotide in its genome.

In yet another embodiment of this aspect of the present invention, the transgene as described in the previous embodiment above may be integrated into the genomic DNA of an avian spermatozoon cell or a precursor cell by an *in vitro* method as, for example, by contacting the spermatozoon cell or a precursor cell with a gene delivery mixture comprising a viral vector, at about or below the avian's body temperature and for an effective period of time such that the transcription unit is incorporated into the genome of the cell; isolating or selecting the genetically modified cell with the aid of a genetic selection marker expressed in the genetically modified cell; transferring the isolated or selected genetically modified cell to a testis of a recipient male avian such that the cell lodges in a seminiferous tubule of the testis and a genetically modified male gamete is produced therein; and breeding the recipient male avian with a female avian of its species such that a transgenic progeny is thereby produced that carries the polynucleotide in its genome.

In yet another embodiment of this aspect of the present invention, the transgene is integrated into the genomic DNA of an avian sperm by restriction enzyme mediated integration (REMI) comprising administering to an avian sperm cell or a precursor sperm cell a gene delivery mixture, wherein a heterologous polynucleotide may encode an immunoglobulin heavy chain variable region, an immunoglobulin heavy chain comprising a variable region and a constant region, an immunoglobulin light chain variable region, an immunoglobulin light chain comprising a variable region and a constant region, a single-chain antibody comprising two linked immunoglobulin variable regions, and the like, such that the heterologous polynucleotide is operatively linked to a promoter sequence such that a transcriptional unit is formed, and where cohesive ends, identical to the cohesive ends characteristic of a DNA cleaved by a given type II restriction endonuclease, have been formed on the heterologous polynucleotide. The heterologous polynucleotide and the type II restriction endonuclease may be transferred into a spermatozoon cell or a precursor cell, thereby incorporating the heterologous polynucleotide into the genome of the spermatozoon cell or the precursor cell, so that a genetically modified male gamete is produced by the male avian. The male avian may then be bred with a female of the same species such that a transgenic progeny is thereby produced that carries the polynucleotide in its genome.

Another aspect of the present invention provides a transgenic avian producing an antibody in an avian egg, wherein the transgenic avian comprises at least one heterologous nucleic acid sequence encoding the polypeptide components of an antibody molecule capable of selectively binding an antigen and wherein antibody is delivered to the white of an avian egg by a female of the avian.

In one embodiment of this aspect of the present invention, the transgenic avian comprises a transcription unit comprising a heterologous nucleotide sequence encoding at least one immunoglobulin polypeptide, a transcription promoter, and a transcriptional terminator operatively linked to the nucleotide sequence encoding at least one immunoglobulin polypeptide.

In another embodiment of this aspect of the present invention, the transgene of the transgenic avian further comprises an internal ribosome entry site (IRES) operatively linked to a nucleotide sequence encoding at least one immunoglobulin polypeptide.

Yet another aspect of the present invention is a transgenic avian egg obtained from a transgenic avian, wherein the egg includes at least one heterologous polynucleotide encoding an antibody capable of selectively binding to an antigen, wherein the white of the avian egg comprises the antibody encoded by the heterologous polynucleotide.

Another aspect of the present invention provides a transgenic avian producing an antibody in an avian serum, wherein the transgenic avian comprises at least one heterologous nucleic acid sequence encoding the polypeptide components of an antibody molecule capable of selectively binding an antigen and wherein antibody is delivered to the serum of the avian.

In one embodiment of this aspect of the present invention, the transgenic avian comprises a transcription unit comprising a heterologous nucleotide sequence encoding at least one immunoglobulin polypeptide, a transcription promoter, and a transcriptional terminator operatively linked to the nucleotide sequence encoding at least one immunoglobulin polypeptide.

In another embodiment of this aspect of the present invention, the transgene of the transgenic avian further comprises an internal ribosome entry site (IRES) operatively linked to a nucleotide sequence encoding at least one immunoglobulin polypeptide.

Yet another aspect of the present invention is a transgenic serum obtained from a transgenic avian, wherein the serum includes at least one heterologous polynucleotide encoding an antibody capable of selectively binding to an antigen, wherein the avian serum comprises the antibody encoded by the heterologous polynucleotide.

Although preferred embodiments of the invention have been described using specific terms, devices, and methods, such description is for illustrative purposes only. The words used are words of description rather than of limitation. It is to be understood that changes and variations may be made by those of ordinary skill in the art without departing from the spirit or the scope of the present invention, which is set forth in the appended claims. In addition, it should be understood that aspects of the various embodiments may be interchanged both in whole or in part. The present invention is further illustrated by the following examples, which are provided by way of illustration and should not be construed as limiting.

### Example 1: Transfection of cultured quail oviduct cells

The oviduct was removed from a Japanese quail *(Coturnix coturnix japonica)* and the magnum portion minced and enzymatically dissociated with 0.8 mg/ml collagenase (Sigma Chemical Co., St. Louis, MO) and 1.0 mg/ml dispase (Roche Molecular Biochemicals, Indianapolis, IN) by shaking and titurating for 30 min at 37°C. The cell suspension was then filtered through sterile surgical gauze, washed three times with F-12 medium (Life Technologies, Grand Island, NY) by centrifugation at 200 x g, and resuspended in OPTIMEM^{™} (Life Technologies) such that the OD₆₀₀ was approximately 2. 300 µl of cell suspension was plated per well of a 24-well dish.

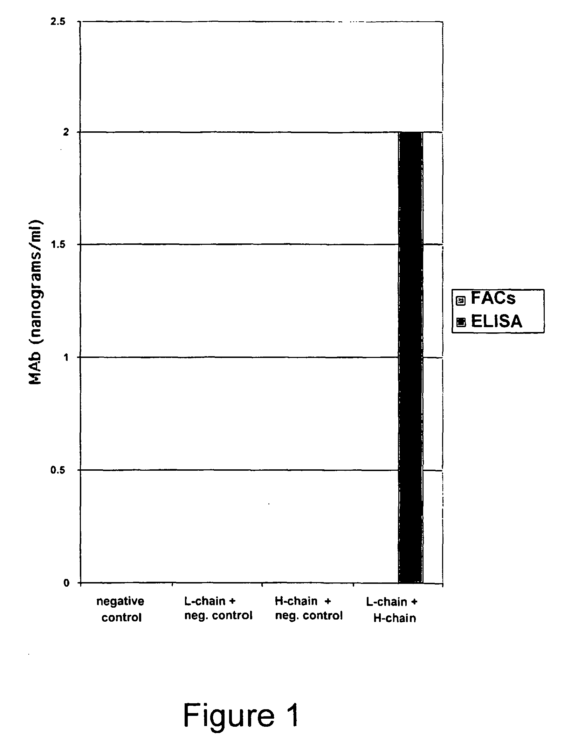

Separate vectors containing a cDNA coding for either the heavy chain or light chain of a human monoclonal antibody against CTLA-4 (WO 01/14424, the contents of which is herein incorporated by reference in its entirety) were provided by an antibody company. For each transfection, 2.5 µl of DMRIE-C liposomes (Life Technologies) and 1 µg of cDNA were preincubated 15 minutes at room temperature in 100 µl of OPTIMEM^{™} and then added to the oviduct cells. Cells with DNA/liposomes were incubated for 5 hours at 37°C in 5% CO₂. Next, 0.75 ml of DMEM (Life Technologies), supplemented with 15% fetal bovine serum (FBS) (Atlanta Biologicals, Atlanta, GA), 2X penicillin/streptomycin (Life Technologies), 10⁻⁶ M insulin (Sigma), 10⁻⁸ M β-estradiol (Sigma), and 10⁻⁷ M corticosterone (Sigma) was added to each well, and incubation continued for 72 hours. Medium was then harvested and centrifuged at 110 x g for 5 minutes. The supernatant was analyzed by ELISA and FACS for antibody content. Referring now to Fig. 1, results indicate that only the cells co-transfected with both heavy chain (p1083) and light chain (p1086) expressed monoclonal antibody detectable by ELISA, however the levels were below detectable limits by FACS.

### Example 2: pCMV-L chain-IRES-H chain (L-IRES-H) Preparation

The pCMV-L-IRES-H vector (designated as pAVIWH-A149.70.1.8) was made by litigating three DNA fragments from three separate plasmids: p1087, pBS-IRES, p1083. The plasmids p1087 and p1083 were obtained as described above in Example 1, while pBS-IRES was obtained from Dr. Peter Mountford (University of Edinburgh). Restriction enzyme digestion of p1087 with *Xba*I and *Eco*RI, followed by alkaline phosphatase treatment, was performed according to standard molecular techniques (Sambrook et al, *supra).* The resulting 6259 base pair (bp) fragment was gel purified by electroelution.

The second plasmid, pBS-IRES, was digested with *Eco*RI and *Nco*I and the resulting 592 bp fragment gel purified as described above. In a similar manner, p1083 was digested with *Nco*I and *Xba*I and the resulting 1500 bp fragment gel purified. All three of the purified DNA fragments were ligated overnight at 16° C in the presence of T4 DNA ligase, used to transform *E. coli* DH5α, and ampicillin resistant colonies were screened by restriction digest. The resulting plasmid, pCMV-L-IRES-H, was purified by QIAGEN prep (Qiagen Inc., Valencia, CA) and used as described in Example 3.

### Example 3: Transfection of cultured chicken whole embryo fibroblasts

To determine if antibody was produced by cells transfected either with heavy and light chain cDNAs on separate plasmids, obtained as described in Example 1, or encoded together on the same plasmid, as described in Example 2, chicken whole embryo fibroblasts (WEFs) were obtained and prepared as follows. Fertile chicken eggs were incubated for approximately 65 hours. Embryos were collected using filter paper rings, then washed three times in phosphate buffered saline with glucose (PBS-G) followed by a wash in calcium- and magnesium-free EDTA (CMF-EDTA). Embryos were then incubated in fresh CMF-EDTA at 4°C with gentle shaking for 30 minutes. CMF-EDTA was removed, and replaced with 0.5% trypsin solution (no EDTA) at 37°C for 3 minutes. Cells were titurated 10 times, then 5% chicken serum was added to inhibit the trypsin reaction. The cell suspension was then added to α-MEM (Life Technologies) supplemented with 2.2 g/l NaHCO₃, 2.52 g/L EPPS, 0.18 g/l D-glucose, 5% FBS, 5% chick serum (heat inactivated at 55°C for 1 hour), 5x10⁻⁵M β-mercaptoethanol, 0.2 mM L-glutamine, 2X penicillin/streptomycin and centrifuged. Cells were resuspended in α-MEM supplemented as described above, and plated on 6-well dishes at a density of 2 x 10⁵ cells per well.

For each transfection, 6 µl of FuGene 6 liposomes (Roche Molecular Biochemicals) and 2 µg of DNA were preincubated 15 min at room temperature in 100 µl of OPTIMEM^{™}, then added to the WEFs. WEFs with DNA/liposomes were incubated 5 hours at 37°C in 5% CO₂. The transfection medium was then removed and replaced with 2 ml of α-MEM supplemented as described above. Medium was removed 72 hours after transfection and centrifuged at 110 x g for 5 minutes.

The supernatants were analyzed for antibody content by ELISA and FACs. Referring now to Fig. 2, results showed that co-transfection of cells with both heavy and light chain plasmids produced monoclonal antibody detected by both ELISA and FACS analysis.

Fig. 3 shows culture results obtained when WEFs are transfected with pCMV-EGFP alone (negative control), cotransfected with p1086 (L-chain) and p1083 (H-chain), or transfected with either 1µg or 2 µg of pCMV-L chain-IRES-H chain (L-IRES-H). ELISA analysis indicates that cells transfected with the vector encoding both cDNAs separated by an IRES element produce detectable antibody. However, antibody production in cells containing the IRES construct is about 10-fold lower than antibody produced by co-transfected cells.

### Example 4: Production of Human Atibody in Chick Serum by Sperm-Mediated Transgenesis

DNA constructs, prepared as described in Examples 1 and 2 above, were also integrated into the chicken genome using sperm-mediated transgenesis (SMT) and shown to express antibody in the serum of the resulting chicks. SMT may involve transfection, electroporation, or incubation of sperm with the desired DNA construct (i.e., the lysozyme promoter controlling expression of heavy and light chains of the MAb) and fertilization of ovum with the treated sperm by artificial insemination or by chicken intracytoplasmic sperm injection (chICSI^{™}).

Liposome complexes were formed with 5 µg each of MluI digested p1086 and p1083 plasmids and 10 µg of LIPTOFECTAMINE in 200 µl of OPTIMEM (Life Technologies). In a separate tube, 100 units of MluI restriction enzyme was mixed with 10 µg of LIPOFECTAMINE in 200 µl of OPTIMEM. Both tubes were incubated at room temperature for 30 minutes, added to 10⁹ freshly-ejaculated sperm from a White Leghorn rooster, and incubated 30 minutes at room temperature. The sperm/liposome/DNA/restriction enzyme mixture was then used to artificially inseminate four White Leghorn hens. On the second and subsequent days following insemination, eggs were collected and incubated at 38°C until hatched.

Serum samples were collected from 40 chicks ranging in age from 3 to 6 weeks old. For each sample, approximately 100 µl of chick blood was collected in heparinized capillary tubes and added to 100 µl of phosphate-buffered saline in a 96-well plate. The plate was centrifuged 5 minutes at 110 x g and approximately 100 µl of supernatant from each well was transferred to 0.5 ml eppendorf tubes for antibody determination. ELISA results showed detectable levels (~2 ng/ml) of human monoclonal antibody in five of the 40 samples.

### Example 5: Generation of Transgenic Chickens Expressing Human Monoclonal Antibodies (MAbs) using a Retroviral Platform

A retroviral vector, based on either avian leukosis virus (ALV) or Moloney murine leukemia virus (MoMLV), will be constructed such that the light (L) and heavy (H) chains of the MAb will be linked by an internal ribosome entry site (IRES) element. Both genes will then be transcriptionally regulated by a promoter such as the cytomegalovirus (CMV) immediate early promoter/enhancer or a promoter that demonstrates tissue specificity for the hen oviduct (i.e. lysozyme promoter, ovalbumin promoter, etc.). The promoter-L chain-IRES-H chain DNA expression cassette will be flanked by the long terminal repeats (LTRs) of the retrovirus. Stage X chicken embryos will be injected with transducing particles containing the above construct to generate transgenic chickens.

Alternatively, the heavy and light chains will be included in separate retroviral vectors and separate lines of transgenic chickens will be generated. Each line will either express the heavy or light chain of the MAb. Once germline transmission of the transgene is established in the two lines, they will be bred to each other in order to express heavy and light chains together to make functional MAbs in the offspring.

### Example 6: Preparation of a Recipient Cytoplast using TPLSM

### Preparation of avian embryo for visualization

Ova were isolated from euthanized hens between 2-4 hours after oviposition of the previous egg. Alternatively, eggs may be isolated from hens whose oviducts have been fistulated (Gilbert & Woodgush, 1963, *J. Reprod. & Fertility* 5: 451-453 and Pander *et al.*, 1989, *Br. Poult. Sci.* 30: 953-7).

Before generating images of the avian early embryo, DNA was incubated with a specific dye according to the following protocol. The albumen capsule was removed and the ovum placed in a dish with the germinal disk facing the top. Remnants of the albumen capsule were removed from the top of the germinal disk. Phosphate buffered saline was added to the dish to prevent drying of the ovum. A cloning cylinder was placed around the germinal disk and 1.0µg/ml of DAPI in PBS was added to the cylinder. Visualization was performed after approximately 15 minutes of incubation.

### Injection of the germinal disk

Preparation of the egg was done as described for incubation. Following removal of the capsule, 10-50 nanoliters of a 0.1 µg/ml solution of DAPI in PBS was injected into the germinal disk using a glass pipette. Visualization was performed approximately 15 minutes after injection.

### Visualization, Nuclear Ablation, and Enucleation

Following incubation, images of the inside of the avian early embryo were generated through the use of TPLSM. The germinal disk was placed under the microscope objective, and the pronuclear structures were searched within the central area of the disk, to a depth of 60µm using low laser power of 3-6 milliwatts at a wavelength of 750 nm.

Once the pronuclear structures were located, they were subjected to laser-mediated ablation. In these experiments, an Olympus 20x/0.5NA (Numerical Aperture) water immersion lens was used. The x and y planes to be ablated were defined with the two photon software, while the z plane (depth) was just under 10µm for this type of objective. Since each pronuclear structure was about 20 µm in diameter, the ablation comprised two steps (2 times 10µm). The focal point was lowered to visualize the remaining of the pronucleus, which was subsequently ablated. The laser power used to ablate the pronuclei was between 30 to 70 milliwatts at a wavelength of 750 nm. For the ablation experiments described above, the image was zoomed by a factor of 4 to 5, giving an area compression of 16-25 fold. Then the power was increased 10-12 fold for a total intensity increase of 160-300 fold compared to the visualization intensity of 3-6 milliwatts. The ablation intensity (power density) is the functional parameter, i.e. the power increase of 10-12 fold results in ablation power of 30-70 milliwatts, but the zoom factor compressed this power into an area 16-25x smaller giving a power density increase of 160-300 fold.

### Example 7: Preparation of a Nuclear Donor Cell and Donor Nucleus Isolation

Fibroblast cells in culture were trypsinized (0.25% Trypsin and 1µM EDTA), centrifuged twice in PBS containing 5% of fetal calf serum (FCS) and placed in a 60 mm plastic dish in PBS containing 5% of FCS. Using the microscope/micromanipulation unit described below, under transmission light, nuclear donors were then isolated by repeated pipetting of the cells, which disrupted the cytoplasmic membrane and released the nucleus from inside the cell.

### Example 8: Preparation of a Reconstructed Zygote

### Injection

A micromanipulation unit, comprising an IM-16 microinjector and a MM-188NE micromanipulator, both from Nikon/Marishige, were adapted to an upright Nikon Eclipse E800. This microscope was adapted to operate under both transmission and reflective light conditions. This unique configuration has allowed us to morphologically examine and prepare (isolate the nuclei, as described above) somatic cells in suspension and to load the injection pipette using dry or water immersion lenses under diascopic illumination or transmitted light. This was followed by prompt localization and positioning of the germinal disk under the microscope and subsequent guided injection of the somatic cells, using dry and long distance lenses under fiber optic as well as episcopic illumination (light coming from the side and through the objectives onto the sample respectively).

### Example 9: Ovum Transfer

At the time of laying, recipient hens were anesthetized by wing vein injection with pentobarbital (0.7 ml of a 68 mg/ml solution). At this time, the infundibulum is receptive to a donor ovum but has not yet ovulated. Feathers were removed from the abdominal area, the area was scrubbed with betadine, and rinsed with 70% ethanol. The bird was placed in a supine position and a surgical drape placed over the bird with the surgical area exposed. An incision, approximately two inches in length, was made beginning at the junction of the sternal rib to the breastbone and running parallel to the breastbone. After cutting through the smooth muscle layers and the peritoneum, the infundibulum was located, externalized and opened using gloved hands and sterile technique. The donor ovum was gently applied to the open infundibulum and allowed to move into the infundibulum, and subsequently into the anterior magnum, by gravity feed. The internalized ovum was placed into the body cavity and the incision closed using interlocking stitches both for the smooth muscle layer and the skin. Recipient hen were returned to their cages and allowed to recover with free access to both feed and water. Eggs laid by the recipient hens were collected the next day, set and hatched 21 days later.

Alternatively, a hen having a fistulated oviduct (Gilbert and Woodgush, *supra* and Pancer *et al., supra)* provides a method for egg collection useful for the enucleation procedure described above. The transfer of a reconstructed embryo to a recipient hen for the production of a hard shell egg (described in Wentworth, 1960, *Poultry Science,* 39: 782-784, *inter alia).* The enucleation technique will be used to obtain ova for recipient cytoplasts and the latter technique to produce recipient hens to be used repeatedly for the transfer of reconstructed embryos.

## Claims

1. A method for the production of a heterologous antibody comprising culturing an avian oviduct cell transfected with at least one expression vector comprising a transcription unit having a nucleotide sequence encoding at least one immunoglobulin polypeptide operably linked to a transcription promoter and a transcriptional terminator under conditions such that said nucleotide sequence is expressed, wherein the cultured avian cell produces an immunoglobulin polypeptide that forms an antibody that selectively binds an antigen or an immunoglobulin polypeptide that, when combined with its cognate light or heavy chain, forms an antibody that selectively binds an antigen.

2. The method of Claim 1, wherein the at least one expression vector further encodes a second immunoglobulin polypeptide and an internal ribosome entry site (IRES).

3. The method of Claim 1, wherein the at least one expression vector is selected from a viral vector, a plasmid vector, or a linear nucleic acid vector.

4. The method of Claim 3, wherein the at least one expression vector is a viral vector selected from the group consisting of avian leukosis virus, adenoviral vectors, transferrin-polylysine enhanced adenoviral vectors, human immunodeficiency virus vectors, lentiviral vectors, Moloney murine leukemia virus-derived vectors or variants thereof.

5. The method of Claim 3, wherein the at least one expression vector is a plasmid vector.

6. The method of Claim 1, wherein the transcriptional promoter of the at least one expression vector is a constitutively active promoter.

7. The method of Claim 6, wherein the transcriptional promoter of the at least one expression vector is a cytomegaloviral promoter.

8. The method of Claim 1, wherein the transcriptional promoter of the at least one expression vector is a tissue-specific promoter.

9. The method of Claim 8, wherein the tissue-specific promoter directs expression in oviduct cells of an avian species.

10. The method of Claim 9, wherein the tissue-specific promoter is selected from the promoters of the genes encoding ovalbumin, lysozyme, ovomucoid, ovotransferrin (conalbumin), and ovomucin.

11. The method of Claim 1, wherein the transcriptional promoter of the at least one expression vector is a regulatable promoter.

12. The method of Claim 1, wherein the transcriptional terminator of the at least one expression vector comprises a region encoding a bovine growth hormone transcriptional terminator.

13. The method of Claim 1, wherein the avian cell is a chicken cell, a turkey cell, a duck cell, a goose cell, a quail cell, a pheasant cell, a ratite cell, an ornamental bird cell or a feral bird cell.

14. The method of claim 1 wherein the oviduct cell is a magnum cell.

15. The method of Claim 1, wherein the immunoglobulin polypeptide is an immunoglobulin heavy chain variable region, an immunoglobulin heavy chain variable region and a constant region, an immunoglobulin light chain variable region, an immunoglobulin light chain variable region and a constant region or a single-chain antibody comprising two linked immunoglobulin variable regions.

16. The method of Claim 1, wherein the immunoglobulin polypeptide has a peptide region for the isolation of the immunoglobulin polypeptide.

17. The method of Claim 1, wherein the immunoglobulin polypeptide encoded by the transcriptional unit of the at least one expression vector is an immunoglobulin heavy chain variable region or a variant thereof.

18. The method of Claim 17, wherein the immunoglobulin heavy chain further comprises a D region, a J region and a C region.

19. The method of Claim 1, wherein the at least one immunoglobulin polypeptide encoded by the transcriptional unit of at least one expression vector is an immunoglobulin light chain variable region or a variant thereof.

20. The method of Claim 19, wherein the immunoglobulin light chain further comprises a J region and a C region.

21. The method of Claim 17, wherein the immunoglobulin polypeptide is a mammalian or an avian immunoglobulin heavy chain polypeptide.

22. The method of Claim 21, wherein the immunoglobulin heavy chain polypeptide comprises at least two domains derived from at least two animal species.

23. The method of Claim 21, wherein the mammal is a human, a mouse, a rat, a rabbit, a goat, a sheep, a cow or a horse, and wherein the avian is a chicken, a turkey, a duck, a goose, a quail, a pheasant, a ratite, an ornamental bird or a feral bird.

24. The method of Claim 1, wherein the immunoglobulin polypeptide is a mammalian or an avian immunoglobulin light chain polypeptide.

25. The method of Claim 24, wherein the immunoglobulin polypeptide comprises at least two domains derived from at least two animal species.

26. The method of Claim 24, wherein the mammal is a human, a mouse, a rat, a rabbit, a goat, a sheep, a cow or a horse, and wherein the avian is a chicken, a turkey, a duck, a goose, a quail, a pheasant, a ratite, and ornamental bird or a feral bird.

27. The method of Claim 1, wherein the immunoglobulin polypeptide encoded by the transcriptional unit of at least one expression vector comprises an immunoglobulin heavy chain variable region, an immunoglobulin light chain variable region, and a linker peptide, and thereby forming a single-chain antibody.

28. The method of Claim 1 wherein the immunoglobulin polypeptide is human.

29. The method of Claim 1 wherein the immunoglobulin polypeptide is humanized.

30. A method as claimed in claim 1 wherein the nucleotide sequence encodes an antibody specific for CTLA4.

31. A method as claimed in claim 30 wherein the antibody is a monoclonal antibody.

32. The method of Claim 30 wherein the avian cell is selected from the group consisting of a chicken cell, a turkey cell, a duck cell, a goose cell, a quail cell, a pheasant cell, a ratite cell, an ornamental bird cell and a feral bird cell.

33. The method of Claim 30 wherein the avian cell is a chicken cell.

34. The method of Claim 30 wherein the antibody is a human antibody.

35. The method of claim 30 wherein the oviduct cell is a magnum cell.

## Patentansprüche

1. Methode für die Herstellung eines heterologen Antikörpers, umfassend Kultivieren einer Vogel-Oviduktzelle, die mit mindestens einem Expressionsvektor transfiziert ist, der eine Transkriptionseinheit umfasst, die eine Nukleotidsequenz aufweist, kodierend für mindestens ein Immunglobulin-Polypeptid, operativ mit einem Transkriptionspromotor und einem transkriptionellen Terminator unter solchen Bedingungen verknüpft, dass die besagte Nukleotidsequenz exprimiert wird, wobei die kultivierte Vogelzelle ein Immunglobulin-Polypeptid herstellt, das einen Antikörper bildet, der selektiv ein Antigen bindet, oder ein Immunglobulin-Polypeptid herstellt, das, wenn kombiniert mit seiner verwandten leichten oder schweren Kette, einen Antikörper bildet, der selektiv ein Antigen bindet.

2. Methode nach Anspruch 1, wobei der mindestens eine Expressionsvektor ferner für ein zweites Immunglobulin-Polypeptid und eine interne Ribosomen-Eintrittsstelle kodiert (Internal Ribosome entry site; IRES).

3. Methode nach Anspruch 1, wobei der mindestens eine Expressionsvektor ausgewählt wird aus einem viralen Vektor, einem Plasmidvektor, oder einem linearen Nukleinsäurevektor.

4. Methode nach Anspruch 3, wobei der mindestens eine Expressionsvektor ein viraler Vektor ist, ausgewählt aus der Gruppe bestehend aus Aviäres Leukosevirus, Adenovirusvektoren, Transferrin-Polylysin verstärkten Adenovirus-Vektoren, humane Immundefizienz Virus Vektoren, Lentivirus Vektoren, Moloney-Maus-Leukämievirus abgeleitete Vektoren oder Varianten davon.

5. Methode nach Anspruch 3, wobei der mindestens eine Expressionsvektor ein Plasmidvektor ist.

6. Methode nach Anspruch 1, wobei der transkriptionelle Promotor des mindestens einen Expressionsvektors ein konstitutiv aktiver Promotor ist.

7. Methode nach Anspruch 6, wobei der transkriptionelle Promotor des mindestens einen Expressionsvektors ein Cytomegalovirus-Promotor ist.

8. Methode nach Anspruch 1, wobei der transkriptionelle Promotor des mindestens einen Expressionsvektors ein gewebespezifischer Promotor ist.

9. Methode nach Anspruch 8, wobei der gewebespezifische Promotor Expression in Oviduktzellen einer Vogelspezies vermittelt.

10. Methode nach Anspruch 9, wobei der gewebespezifische Promotor aus den Promotoren der Gene ausgewählt wird, die für Ovalbumin, Lysozym, Ovomucoid, Ovotransferrin (Conalbumin) und Ovomucin kodieren.

11. Methode nach Anspruch 1, wobei der transkriptionelle Promotor des mindestens einen Expressionsvektors ein regulierbarer Promotor ist.

12. Methode nach Anspruch 1, wobei der transkriptionelle Terminator des mindestens einen Expressionsvektors eine Region umfasst, die für einen transkriptionellen Terminator eines Rinderwachstumshormons kodiert.

13. Methode nach Anspruch 1, wobei die Vogelzelle eine Huhnzelle, eine Truthahnzelle, eine Entenzelle, eine Gänsezelle, eine Wachtelzelle, eine Fasanenzelle, eine Laufvogelzelle, eine Ziervogelzelle oder eine Wildvogelzelle ist.

14. Methode nach Anspruch 1, wobei die Oviduktzelle eine Magnumzelle ist.

15. Methode nach Anspruch 1, wobei das Immunglobulin-Polypeptid die variable Region der schweren Kette eines Immunglobulins, die variable Region der schweren Kette eines Immunglobulins und eine konstante Region, die variable Region der leichten Kette eines Immunglobulins, die variable Region der leichten Kette eines Immunglobulins und eine konstante Region, oder ein einzelkettiger Antikörper, umfassend zwei verknüpfte variable Regionen eines Immunglobulins, ist.

16. Methode nach Anspruch 1, wobei das Immunglobulin-Polypeptid eine Peptidregion für die Isolierung des Immunglobulin-Polypeptids aufweist.

17. Methode nach Anspruch 1, wobei das Immunglobulin-Polypeptid, das von der transkriptionellen Einheit des mindestens einen Expressionsvektors kodiert wird, die variable Region der schweren Kette eines Immunglobulins oder eine Variante davon ist.

18. Methode nach Anspruch 17, wobei die schwere Kette des Immunglobulins ferner eine D Region, eine J Region und eine C Region umfasst.

19. Methode nach Anspruch 1, wobei das mindestens eine Immunglobulin-Polypeptid, das von der transkriptionellen Einheit des mindestens einen Expressionsvektors kodiert wird, die variable Region der leichten Kette eines Immunglobulins oder eine Variante davon ist.

20. Methode nach Anspruch 19, wobei die leichte Kette des Immunglobulins ferner eine J Region und eine C Region umfasst.

21. Methode nach Anspruch 17, wobei das Immunglobulin-Polypeptid die schwere Kette eines Immunglobulins aus einem Säuger oder einem Vogel kodierendes Polypeptid ist.

22. Methode nach Anspruch 21, wobei die schwere Kette des Immunglobin-Polypeptids mindestens zwei Domänen umfasst, die aus mindestens zwei Tierspezies abgeleitet sind.

23. Methode nach Anspruch 21, wobei der Säuger ein Mensch, eine Maus, eine Ratte, ein Kaninchen, eine Ziege, ein Schaf, eine Kuh oder ein Pferd ist, und wobei der Vogel ein Huhn, ein Truthahn, eine Ente, eine Gans, eine Wachtel, ein Fasan, ein Laufvogel, ein Ziervogel, oder ein Wildvogel ist.

24. Methode nach Anspruch 1, wobei das Immunglobulin-Polypeptid die leichte Kette eines Immunglobulins aus einem Säuger oder einem Vogel kodierendes Polypeptid ist.

25. Methode nach Anspruch 24, wobei das Immunglobulin-Polypeptid mindestens zwei Domänen umfasst, die aus mindestens zwei Tierspezies abgeleitet sind.

26. Methode nach Anspruch 24, wobei der Säuger ein Mensch, eine Maus, eine Ratte, ein Kaninchen, eine Ziege, ein Schaf, eine Kuh oder ein Pferd ist, und wobei der Vogel ein Huhn, ein Truthahn, eine Ente, eine Gans, eine Wachtel, ein Fasan, ein Laufvogel, ein Ziervogel, oder ein Wildvogel ist.

27. Methode nach Anspruch 1, wobei das Immunglobulin-Polypeptid, das von der transkriptionellen Einheit des mindestens einen Expressionsvektors kodiert wird, die variable Region der schweren Kette eines Immunglobulins, die variable Region der leichten Kette eines Immunglobulins und ein Bindepeptid umfasst, und dadurch einen einzelkettigen Antikörper bildet.

28. Methode nach Anspruch 1, wobei das Immunglobulin-Polypeptid menschlich ist.

29. Methode nach Anspruch 1, wobei das Immunglobulin-Polypeptid humanisiert ist.

30. Methode wie in Anspruch 1 beansprucht, wobei die Nukleotidsequenz einen Antikörper kodiert, der spezifisch für CTLA4 ist.

31. Methode wie in Anspruch 30 beansprucht, wobei der Antikörper ein monoklonaler Antikörper ist.

32. Methode nach Anspruch 30, wobei die Vogelzelle ausgewählt wird aus der Gruppe, die aus einer Huhnzelle, einer Truthahnzelle, einer Entenzelle, einer Gänsezelle, einer Wachtelzelle, einer Fasanenzelle, einer Laufvogelzelle, einer Ziervogelzelle und einer Wildvogelzelle besteht.

33. Methode nach Anspruch 30, wobei die Vogelzelle eine Huhnzelle ist.

34. Methode nach Anspruch 30, wobei der Antikörper ein menschlicher Antikörper ist.

35. Methode nach Anspruch 30, wobei die Oviduktzelle eine Magnumzelle ist.

## Revendications

1. Procédé de production d'un anticorps hétérologue comprenant la culture d'une cellule aviaire d'oviducte transfectée avec au moins un vecteur d'expression comprenant une unité de transcription ayant une séquence de nucléotides codant au moins un polypeptide d'immunoglobuline lié de façon opérationnelle à un promoteur de transcription et à un terminateur de transcription dans des conditions telles que ladite séquence de nucléotides est exprimée, dans lequel la cellule aviaire en culture produit un polypeptide d'immunoglobuline formant un anticorps qui se lie sélectivement à un antigène ou un polypeptide d'immunoglobuline qui, quand il est combiné à sa chaîne lourde ou légère apparentée, forme un anticorps qui se lie sélectivement à un antigène.

2. Procédé selon la revendication 1, dans lequel le au moins un vecteur d'expression code en outre un second polypeptide d'immunoglobuline et un site d'entrée interne du ribosome (IRES).

3. Procédé selon la revendication 1, dans lequel le au moins un vecteur d'expression est choisi parmi un vecteur viral, un vecteur plasmide ou un vecteur d'acide nucléique linéaire.

4. Procédé selon la revendication 3, dans lequel le au moins un vecteur d'expression est un vecteur viral choisi dans le groupe constitué par le virus de la leucose aviaire, des vecteurs adénoviraux, des vecteurs adénoviraux améliorés par la transferrine-polylysine, des vecteurs dérivés du virus de l'immunodéficience humaine, des vecteurs lentiviraux, des vecteurs dérivés du virus de la leucémie murine de Moloney ou leurs variants.

5. Procédé selon la revendication 3, dans lequel le au moins un vecteur d'expression est un vecteur plasmide.

6. Procédé selon la revendication 1, dans lequel le promoteur de transcription du au moins un vecteur d'expression est un promoteur constitutivement actif.

7. Procédé selon la revendication 6, dans lequel le promoteur de transcription du au moins un vecteur d'expression est un promoteur de cytomégalovirus.

8. Procédé selon la revendication 1, dans lequel le promoteur de transcription du au moins un vecteur d'expression est un promoteur spécifique d'un tissu.

9. Procédé selon la revendication 8, dans lequel le promoteur spécifique d'un tissu dirige l'expression dans une cellule d'oviducte d'une espèce aviaire.

10. Procédé selon la revendication 9, dans lequel le promoteur spécifique d'un tissu est choisi parmi les promoteurs des gènes codant l'ovalbumine, le lysozyme, l'ovomucoïde, l'ovotransferrine (conalbumine) et l'ovomucine.

11. Procédé selon la revendication 1, dans lequel le promoteur de transcription du au moins un vecteur d'expression est un promoteur pouvant être régulé.

12. Procédé selon la revendication 1, dans lequel le terminateur de transcription du au moins un vecteur d'expression comprend une région codant un terminateur de transcription de l'hormone de croissance bovine.

13. Procédé selon la revendication 1, dans lequel la cellule aviaire est une cellule de poulet, une cellule de dinde, une cellule de canard, une cellule d'oie, une cellule de caille, une cellule de faisan, une cellule de ratite, une cellule d'oiseau ornemental ou une cellule d'oiseau féral.

14. Procédé selon la revendication 1, dans lequel la cellule d'oviducte est une cellule du magnum.

15. Procédé selon la revendication 1, dans lequel le polypeptide d'immunoglobuline est une région variable de la chaîne lourde de l'immunoglobuline, une région constante et une région variable de la chaîne lourde de l'immunoglobuline, une région variable de la chaîne légère de l'immunoglobuline, une région constante et une région variable de la chaîne légère de l'immunoglobuline ou un anticorps monocaténaire comprenant deux régions variables liées de l'immunoglobuline..

16. Procédé selon la revendication 1, dans lequel le polypeptide d'immunoglobuline possède une région peptidique pour l'isolement du polypeptide d'immunoglobuline.

17. Procédé selon la revendication 1, dans lequel le polypeptide d'immunoglobuline codé par l'unité de transcription du au moins un vecteur d'expression est une région variable de la chaîne lourde de l'immunoglobuline ou un variant de celle-ci.

18. Procédé selon la revendication 17, dans lequel la chaîne lourde de l'immunoglobuline comprend en outre une région D, une région J et une région C.

19. Procédé selon la revendication 1, dans lequel le au moins un polypeptide d'immunoglobuline codé par l'unité de transcription d'au moins un vecteur d'expression est une région variable de la chaîne légère de l'immunoglobuline ou un variant de celle-ci.

20. Procédé selon la revendication 19, dans lequel la chaîne légère de l'immunoglobuline comprend en outre une région J et une région C.

21. Procédé selon la revendication 17, dans lequel le polypeptide d'immunoglobuline est un polypeptide de chaîne lourde d'immunoglobuline aviaire ou de mammifère.

22. Procédé selon la revendication 21, dans lequel le polypeptide de chaîne lourde d'immunoglobuline comprend au moins deux domaines dérivant au moins de deux espèces animales.

23. Procédé selon la revendication 21, dans lequel le mammifère est un humain, une souris, un rat, un lapin, une chèvre, un mouton, une vache ou un cheval, et dans lequel l'animal aviaire est un poulet, une dinde, un canard, une oie, une caille, un faisan, un ratite, un oiseau ornemental ou un oiseau féral.

24. Procédé selon la revendication 1, dans lequel le polypeptide d'immunoglobuline est un polypeptide de chaîne légère d'immunoglobuline aviaire ou de mammifère.

25. Procédé selon la revendication 24, dans lequel le polypeptide d'immunoglobuline comprend au moins deux domaines dérivant au moins de deux espèces animales.

26. Procédé selon la revendication 24, dans lequel le mammifère est un humain, une souris, un rat, un lapin, une chèvre, un mouton, une vache ou un cheval, et dans lequel l'animal aviaire est un poulet, une dinde, un canard, une oie, une caille, un faisan, un ratite, un oiseau ornemental ou un oiseau féral.

27. Procédé selon la revendication 1, dans lequel le polypeptide d'immunoglobuline codé par l'unité de transcription d'au moins un vecteur d'expression comprend une région variable de la chaîne lourde de l'immunoglobuline, une région variable de la chaîne légère de l'immunoglobuline et un peptide lieur, ce qui forme ainsi un anticorps monocaténaire.

28. Procédé selon la revendication 1, dans lequel le polypeptide d'immunoglobuline est humain.

29. Procédé selon la revendication 1, dans lequel le polypeptide d'immunoglobuline est humanisé.

30. Procédé selon la revendication 1, dans lequel la séquence de nucléotides code un anticorps spécifique du CTLA4.

31. Procédé selon la revendication 30, dans lequel l'anticorps est un anticorps monoclonal.

32. Procédé selon la revendication 30, dans lequel la cellule aviaire est choisie dans le groupe constitué par une cellule de poulet, une cellule de dinde, une cellule de canard, une cellule d'oie, une cellule de caille, une cellule de faisan, une cellule de ratite, une cellule d'oiseau ornemental ou une cellule d'oiseau féral.

33. Procédé selon la revendication 30, dans lequel la cellule aviaire est une cellule de poulet.

34. Procédé selon la revendication 30, dans lequel l'anticorps est un anticorps humain.

35. Procédé selon la revendication 30, dans lequel la cellule d'oviducte est une cellule de magnum.
